# EUROPÄISCHE PATENTSCHRIFT

(11) **EP 0 810 989 B1**
(45) Veröffentlichungstag und Bekanntmachung des Hinweises auf die Patenterteilung: **04.08.1999**
(21) Anmeldenummer: 96902992.5
(22) Anmeldetag: 21.02.1996
(51) Int. Cl.: C07C 229/76, A61K 49/00, A61K 31/195, C07C 229/16, C07C 229/22, C07C 233/83, C07C 237/06

(54) **NEUARTIG SUBSTITUIERTE DTPA-DERIVATE, DEREN METALLKOMPLEXE, DIESE KOMPLEXE ENTHALTENDE PHARMAZEUTISCHE MITTEL, DEREN VERWENDUNG IN DER DIAGNOSTIK UND THERAPIE, SOWIE VERFAHREN ZUR HERSTELLUNG DER KOMPLEXE UND MITTEL**
NOVELLY SUBSTITUTED DTPA DERIVATIVES, METAL COMPLEXES THEREOF, PHARMACEUTICALS CONTAINING THESE COMPLEXES, DIAGNOSTIC AND THERAPEUTIC USES THEREOF AND METHODS FOR PREPARING THE COMPLEXES AND PHARMACEUTICALS
DERIVES DE DTPA A SUBSTITUTION NOUVELLE, LEURS COMPLEXES METALLIQUES, PRODUITS PHARMACEUTIQUES CONTENANT CES COMPLEXES, LEURS USAGES DIAGNOSTIQUE ET THERAPEUTIQUE, AINSI QUE PROCEDES POUR PREPARER CES COMPLEXES ET PRODUITS PHARMACEUTIQUES

(30) Priorität: 21.02.1995 DE 19507820
(43) Veröffentlichungstag der Anmeldung: 10.12.1997
(73) Patentinhaber: SCHERING AKTIENGESELLSCHAFT, 13342 Berlin (DE)
(72) Erfinder: KRAUSE, Werner, D-13505 Berlin (DE); MAIER, Franz-Karl, D-13467 Berlin (DE); BAUER, Michael, D-13503 Berlin (DE); SCHUHMANN-GIAMPIERI, Gabriele, D-12203 Berlin (DE); PRESS, Wolf-Rüdiger, D-12103 Berlin (DE); MUSCHICK, Peter, D-16321 Ladeburg (DE)
(86) Internationale Anmeldenummer: EP9600734
(87) Internationale Veröffentlichungsnummer: WO9626180

(56) Entgegenhaltungen:
- EP-A- 0 071 564
- EP-A- 0 230 893
- EP-A- 0 367 223
- EP-A- 0 405 704
- EP-A- 0 450 742
- J. ORG. CHEM. (JOCEAH,00223263);93; VOL.58 (5); PP.1151-8, UNIV. CALIFORNIA;DEP. CHEM.; BERKELEY; 94720; CA; USA (US), XP002002644 WILLIAMS M A ET AL: "Synthesis of enantiomerically pure diethylenetriaminepentaacetic acid analogs. L-Phenylalanine as the educt for substitution at the central acetic acid"

## Beschreibung

Die Erfindung betrifft die in den Ansprüchen gekennzeichneten Gegenstände, das heißt neuartig substituierte DTPA-Derivate, deren Metallkomplexe, diese Komplexe enthaltende pharmazeutische Mittel, deren Verwendung in der Diagnostik und Therapie sowie Verfahren zur Herstellung der Komplexe und Mittel.

Kontrastmittel sind unentbehrliche Hilfsmittel in der modernen Diagnostik; so wären viele Erkrankungen ohne den Einsatz von Kontrastmitteln nicht diagnostizierbar. Kontrastmittel finden in allen Bereichen der Diagnostik wie z.B. der Röntgen-, der Radio- oder der Ultraschalldiagnostik oder der magnetischen Resonanztomographie Verwendung.

Die Wahl der jeweils bevorzugten Methode richtet sich u.a. nach der diagnostischen Fragestellung, wird aber auch durch die dem Mediziner jeweils zur Verfügung stehenden apparativen Möglichkeiten bestimmt. So hat insbesondere die Kernspintomographie aufgrund des erheblichen technischen und damit verbundenen hohen Kostenaufwands noch nicht die weite Verbreitung der anderen Methoden, wie z.B. röntgendiagnostischer Methoden gefunden.

Auch die Wahl des geeigneten Kontrastmittels variiert in Abhängigkeit der jeweiligen Fragestellung. So wird die Eignung des Kontrastmittels für eine bestimmte Aufgabe nicht zuletzt durch sein Anreicherungs- und Verteilungsverhalten im Organismus bestimmt.

Obgleich sowohl auf der gerätetechnischen als auch auf der Kontrastmittelseite große Fortschritte erzielt worden sind, stehen noch nicht für alle Fragestellungen befriedigende Lösungen zur Verfügung.

So gibt es für die verschiedenen bildgebenden Verfahren nicht für alle Indikationen die geeigneten Kontrastmittel. Insbesondere steht bis heute kein geeignetes Röntgenkontrastmittel für die Leberdiagnostik zur Verfügung.

In der Röntgendiagnostik haben sich im wesentlichen Kontrastmittel auf der Basis von Triiodbenzol durchsetzen können, da diese Verbindungen eine hohe Röntgendichte, eine geringe allgemeine und lokale Toxizität aufweisen und sehr gut wasserlöslich sind.

Derartige Verbindungen werden z.B. in der EP 0 105 752, EP 0 015 867 beschrieben. Diese zeigen jedoch keine, für eine Bildgebung ausreichende Anreicherung in der Leber.

Der schattengebende Effekt eines Röntgenkontrastmittels ist im wesentlichen von der Größe des Masseschwächungskoeffizienten der in der Verbindung enthaltenden Elemente im diagnostischen Strahlenbereich abhängig. Neben iodhaltigen Verbindungen sind auch Komplexe von Metallen höherer Ordnungszahl als Röntgenkontrastmittel geeignet. Physiologisch verträgliche Komplexverbindungen dieser Metalle finden bereits im Bereich der NMR-Diagnostik breite Anwendung. Es handelt sich dabei im allgemeinen um Metallkomplexe, wie sie z.B. in der EP 0 071 564 beschrieben werden.

Die in EP 0 230 893, J. Org. chem. Vol. 58, 1151 - 8 (1993) und EP 0 367 223 beschriebenen Metallkomplexe werden von der vorliegenden Erfindung nicht umfaßt. Es findet sich in diesen Referenzen kein Hinweis auf ihre mögliche Verwendung als Röntgenkontrastmittel.

Die WO 93/16375 beschreibt Metallkomplexe, die über Amidbindungen an iodsubstituierte Aromaten geknüpft sind. Diese Verbindungen sollen es mit nur einer Applikation des Kontrastmittels erlauben sowohl NMR- als auch Röntgen-Untersuchungen durchzuführen. Eine Kombination der beiden bildgebenden Verfahren ist in vielen Fällen für eine differenzierte Darstellung und eine zuverlässige Bestimmung bestimmter Erkrankungen von Vorteil. Diese Verbindungen sollen insbesondere für die Angiographie geeignet sein. Wie die Nacharbeitung der Herstellungsbeispiele ergab, zeigen die Verbindungen jedoch keine für Röntgenuntersuchungen ausreichende Anreicherung im Bereich der Leber.

Leberspezifische NMR-Kontrastmittel werden in der EP 0 405 704 beschrieben. Diese sollten wegen des Metallgehaltes in den Komplexen prinzipiell auch für die Röntgendiagnostik geeignet sein. Eine Nacharbeitung der experimentellen Beispiele zeigte im Tierversuch selbst bei Verabreichung einer hohen Dosis (Konz.: 1 mol/l, Dosis: 0,5 mmol Gd/kg intravenös) keine ausreichende Kontrastierung der Leber in der Röntgenaufnahme. Ein ausreichender bildgebender Effekt in der Röntgendiagnostik wird erst bei einer Dosis erreicht, bei der der Sicherheitsabstand auf ein nicht mehr vertretbares Maß reduziert ist.

Aufgabe der vorliegenden Erfindung war es daher, sehr gut verträgliche und wasserlösliche Kontrastmittel sowie ein möglichst einfaches Verfahren zu ihrer Herstellung zur Verfügung zu stellen, die für die NMR-, Röntgen- und Radiodiagnostik oder Radiotherapie - insbesondere für die Röntgendiagnostik der Leber - geeignet sind.

Diese Aufgabe wird durch die in den Patentansprüchen gekennzeichneten Stoffe, Mittel, Herstellungsverfahren und Verwendungen gelöst.

Es wurde gefunden, daß Metallkomplexe der allgemeinen Formel I 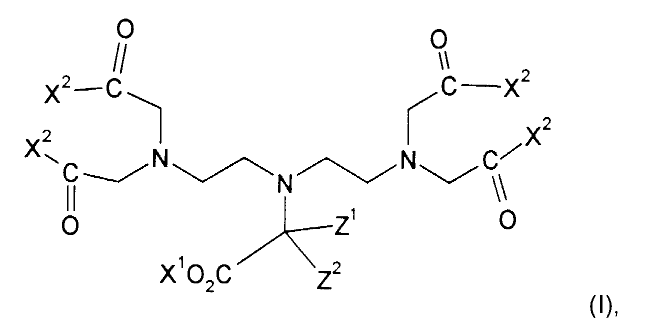 worin
- X¹: unabhängig voneinander für ein Wasserstoffatom oder ein Metallionenäquivalent eines Elements der Ordnungszahlen 20 - 32, 39 - 51 oder 57 - 83 steht,
- X²: unabhängig voneinander für eine Gruppe
O-X¹ mit X¹ in der oben angegebenen Bedeutung oder
N(R¹)R² worin
R¹, R² unabhängig voneinander für ein Wasserstoffatom oder für eine gesättigte oder ungesättigte, verzweigte oder geradkettige C₁-C₂₀-Kette steht, wobei die Kette oder Teile der Kette eine zyklische oder bizyklische Einheit formen können, die durch null bis drei Sauerstoff- und/oder Schwefelatome und/oder null bis drei Sulfoxy- und/oder Sulfonogruppen unterbrochen ist und durch null bis sechs Phenyl-, Pyridyl-, R³S-, R³OOC- und/oder R³O-Gruppen substituiert ist, 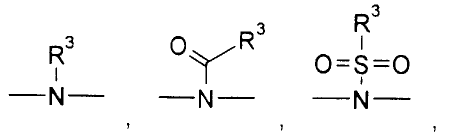 die weiterhin null bis drei 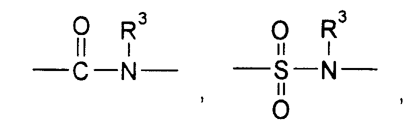 Carbonyl- und/oder Thiocarbonylgruppen enthält,
wobei gegebenenfalls vorhandene aromatische Gruppen ein- bis dreifach unabhängig voneinander durch R³O₂C-, 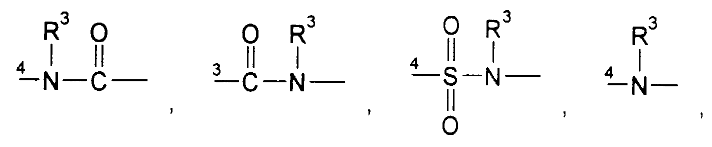 O₂N-, R³O- und/oder R⁴-Gruppen substituiert sein können oder
R¹ und R² zusammen, unter Einbeziehung des gemeinsamen Amid-Stickstoffatomes einen vier- bis achtgliedrigen Ring bilden, der zwei weitere Sauerstoffatome und/oder zwei Carbonylgruppen enthalten kann,
- Z¹: für eine gesättigte oder ungesättigte, verzweigte oder geradkettige C₁-C₂₀-Kette steht, wobei die Kette oder Teile der Kette eine zyklische oder bizyklische Einheit formen können,
die durch null bis drei Schwefelatome und/oder null bis drei Sulfoxy- und/oder Sulfonogruppen unterbrochen ist und durch null bis sechs Phenyl-, Pyridyl-, R³S- und/oder R³OOC-Gruppen substituiert ist, 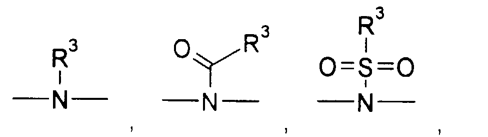 die weiterhin null bis drei 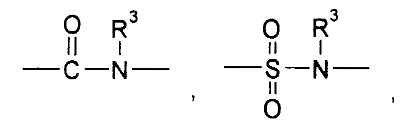 Carbonyl- und/oder Thiocarbonylgruppen enthält,
wobei gegebenenfalls vorhandene aromatische Gruppen ein- oder mehrfach unabhängig voneinander durch R³O₂C-, 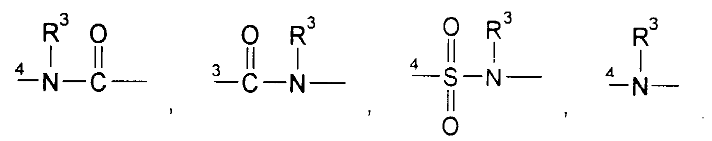 R³O- und/oder R⁴-Gruppen substituiert sein können,
- Z²: für ein Wasserstoffatom oder für eine gesättigte oder ungesättigte, verzweigte oder geradkettige C₁-C₂₀-Kette steht, wobei die Kette oder Teile der Kette eine zyklische oder bizyklische Einheit formen können,
die durch null bis drei Sauerstoff- und/oder Schwefelatome und/oder null bis drei Sulfoxy- und/oder Sulfonogruppen unterbrochen ist und
durch null bis sechs Phenyl-, Pyridyl-, R³S-, R³OOC- und/oder R³O-Gruppen substituiert ist, 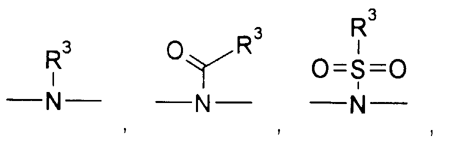 die weiterhin null bis drei 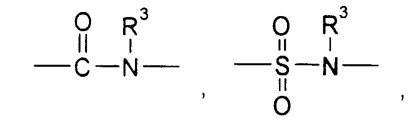 Carbonyl- und/oder Thiocarbonylgruppen enthält,
wobei gegebenenfalls vorhandene aromatische Gruppen ein- bis dreifach unabhängig voneinander durch R³O₂C-, 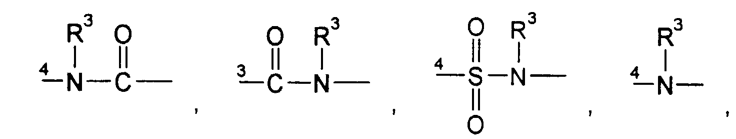 R³O- und/oder R³-Gruppen substituiert sein können,
- R³: unabhängig voneinander für ein Wasserstoffatom, einen Phenylrest oder einen geradkettigen, verzweigten oder zyklischen C₁-C₆-Rest steht, der durch null bis zwei Sauerstoffatome und/oder null bis zwei Phenylengruppen unterbrochen ist und mit null bis drei HO-, HOOC- und/oder null bis zwei Phenylresten substituiert ist,
- R⁴: unabhängig voneinander für einen Phenylrest oder einen geradkettigen, verzweigten oder zyklischen C₁-C₆-Rest steht, der durch null bis zwei Sauerstoffatome und/oder null bis zwei Phenylengruppen unterbrochen ist und mit null bis drei HO- HOOC- und/oder null bis zwei Phenylresten substituiert ist,

wobei falls Z² für ein Wasserstoffatom steht der Rest Z¹ nicht für einen unsubstituierten C₆-C₁₀-Arylrest steht
oder Z¹ und Z² zusammen unter Einbeziehung des gemeinsamen α-Kohlenstoffatoms einen drei- bis achtgliedrigen Ring oder einen Bizyklus mit sieben bis 15 Kohlenstoffatomen formen,
wobei freie, zur Komplexierung nicht herangezogene Carbonsäuregruppen der erfindungsgemäßen Verbindungen der allgemeinen Formel I auch in Form ihrer Salze mit physiologisch verträglichen anorganischen und/oder organischen Kationen vorliegen können,
hervorragend zur Herstellung von Kontrastmitteln für die Röntgen- und/oder NMR-diagnostik, bevorzugt von Kontrastmitteln für die Röntgendiagnostik, insbesondere für die Röntgendiagnostik der Leber, der Gallengänge und der Galle geeignet sind.

Die Erfindung betrifft daher die Verbindungen der allgemeinen Formel I.

Verbindungen der allgemeinen Formel I bei denen alle vorkommenden Reste X¹ die Bedeutung von Wasserstoffatomen haben werden als Komplexbildner bezeichnet. Verbindungen der allgemeinen Formel I bei denen mindestens einer der mit X¹ bezeichneten Reste die Bedeutung eines Metallionenäquivalentes haben, werden als Komplexe bezeichnet.

Ist der erfindungsgemäße Metallkomplex zur Herstellung von Mitteln für die Röntgendiagnostik bestimmt, so muß sich das Zentralion von einem Element höherer Ordnungszahl ableiten, um eine ausreichende Absorption der Röntgenstrahlen zu erzielen. Es wurde gefunden, daß für diesen Zweck Elemente der Ordnungszahlen 57 - 83 geeignet sind. Besonders geeignet sind Komplexe der Elemente Lanthan, Gadolinium, Terbium, Dysprosium, Holmium, Erbium, Ytterbium, Lutetium, Wismut und Hafnium.

Ist der erfindungsgemäße Metallkomplex zur Herstellung von Mitteln für die NMR-diagnostik bestimmt, so muß das Zentralion paramagnetisch sein. Es wurde gefunden, daß für diesen Zweck insbesondere das Chrom(III)-, Eisen(II)-, Cobalt(II)-, Nickel(II)-, Kupfer(II)-, Praseodym(III)-, Neodym(III)-, Samarium(III)- und das Ytterbium(III)ion geeignet sind. Besonders bevorzugt sind Komplexe der lonen Gadolinium(III), Terbium(III), Dysprosium(III), Holmium(III), Erbium(III), Eisen(III) und Mangan(II).

Ist der erfindungsgemäße Metallkomplex zur Herstellung von Mitteln für die Nuklearmedizin bestimmt, so muß das Zentralion radioaktiv sein sein. Geeignet sind zum Beispiel die Radioisotope der Elemente Kupfer, Kobalt, Gallium, Germanium, Yttrium, Strontium, Technetium, Indium, Ytterbium, Gadolinium, Samarium, Silber, Gold, Rhenium, Wismut und Iridium.

Die erfindungsgemäßen Verbindungen können als Gruppen der Formel -C(=O)X² Carboxylate (-CO₂X¹) oder Carbonsäureamide (-C(=O)N(R¹)R²) enthalten. Als Reste R¹ und R² seien beispielsweise genannt Wasserstoffatome, geradkettige und verzweigte, gegebenenfalls durch 1-3 Sauerstoffatome unterbrochene und/oder durch 1-3 Carboxygruppen substituierte C₁-C₂₀-Alkylreste sowie Phenyl- und Benzylreste.

Bevorzugte Reste R¹ und R² sind Wasserstoffatome Methyl-, Benzyl- und/oder C₁-C₁₁-Carboxyalkyreste.

Die Reste R¹ und R² können auch gemeinsam unter Einbeziehung des Amid-Stickstoffatomes, dessen Substituenten sie sind, einen vier- bis achtgliedrigen Ring bilden, der null bis zwei weitere Sauerstoffatome und/oder null bis zwei weitere Carbonyl- oder Sulfonylgruppen enthalten kann. Stehen R¹ und R² gemeinsam für ein Ringsystem, so ist der Morpholinring oder der S,S-Dioxothiomorpholinring bevorzugt.

Als Gruppe Z¹ tragen die erfindungsgemäßen Verbindungen geradkettige oder verzweigte, gesättigte oder ungesättigte C₁-C₂₀-Ketten, die durch 0 bis drei Schwefelatome und/oder null bis drei Sulfoxy- und/oder Sulfonogruppen unterbrochen sein können. Die Kette kann auch zyklische oder bizyklische Einheiten, wie Phenyl-, Phenylen, Naphthyl-, Naphthylen-, Adamantylreste aufweisen. Sie kann bis zu sechs Carboxy-, Carboxyalkyl-, Phenyl- oder Pyridylsubstituenten tragen. Sollte die Kette aromatische Gruppen aufweisen, können diese ihrerseits Hydroxy-, Alkoxy, Carboxy-, Carboxyalkyl-, Nitro-, Amino-, Acylamino- und/oder Carbamidsubstituenten tragen.

Als Reste Z¹ seien beispielhaft genannt: Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonadecyl-, Icosyl-, Cyclopentyl-, Cyclohexyl-, Cycloheptyl-, Cyclooctyl-, Cyclopentanonyl-, 2-Ethyl-hexyl-, Phenyl-, Benzyl- und/oder Naphtylreste sowie Reste der Formeln

CH₂C₆H₄-CH₃, CH₂C₆H₄-OC₂H₅, CH₂C₆H₄-OCH₃,

CH₂C₆H₄-OCH₂C₆H₅, CH₂C₆H₄CH₂C₆H₅, CH₂C₆H₄C₄H₉,

CH₂C₆H₄-OC₃H₇, CH₂C₆H₄C₃H₇, CH₂C₆H₄-OC₄H₉,

(CH₂)₁₋₂-S-CH₂C₆H₅, (CH₂)₁₋₂-S-CH₂C₆H₄CH₃,

(CH₂)₁₋₂S-CH₂C₆H₄C₂H₅, (CH₂)₁₋₂S-CH₂C₆H₄C₃H₇,

(CH₂)₁₋₂S-CH₂C₆H₄C₄H₉, (CH₂)₁₋₂S-CH₂C₆H₄C₆H₅,

(CH₂)₁₋₂-S-CH₂C₆H₄-OCH₃, (CH₂)₁₋₂S-CH₂C₆H₄-OC₂H₅,

(CH₂)₁₋₂S-CH₂C₆H₄-OC₃H₇, (CH₂)₁₋₂S-CH₂C₆H₄-OC₄H₉,

(CH₂)₁₋₂S-CH₂C₆H₄OC₆H₅, CO₂H, (CH₂)₁₋₁₉CO₂H,

(CH₂)₄NHC(O)C₆H₅

und

(CH₂)₄NHC(O)CH₂CH₂CO₂H.

Bevorzugte Gruppen Z¹ sind geradkettige Alkylreste mit 6-20 Kohlenstoffatomen, sowie Reste der Formeln

-(CH₂)ₙCOOH,

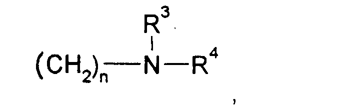

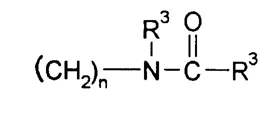

und 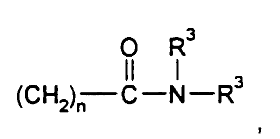 worin n für die Ziffern 1 bis 19 steht und R³ unabhängig voneinander für ein Wasserstoffatom, einen Phenylrest oder einen geradkettigen, verzweigten oder zyklischen C₁-C₆-Rest steht, der durch null bis zwei Sauerstoffatome und/oder null bis zwei Phenylengruppen unterbrochen ist und mit null bis drei HO-, HOOC- und/oder null bis zwei Phenylresten substituiert ist.

R⁴ steht unabhängig voneinander für einen Phenylrest oder einen geradkettigen, verzweigten oder zyklischen C₁-C₆-Rest, der durch null bis zwei Sauerstoffatome und/oder null bis zwei Phenylengruppen unterbrochen ist und mit null bis drei HO-, HOOC- und/oder null bis zwei Phenylresten substituiert ist.

Enthält der Rest Z¹ Carboxylgruppen, so können diese auch als Salze mit einem oder mehreren physiologisch verträglichen Kationen vorliegen. Weiterhin können derartige Carboxylgruppen auch als Elektronendonor für das Zentralatom des Komplexes dienen.

Ebenfalls bevorzugte Gruppen Z¹sind Reste der Formel

-(CH₂)ₚ(C₆H₄)-C_{q}H_{(2q+1)},

worin p und q für die Ziffern eins bis fünf stehen.

Besonders bevorzugte Gruppen Z¹sind weiterhin Reste der Formeln

-(CH₂)ₘ(C₆H₄)-R⁵,

worin R⁵ für einen Butyl-, Phenyl- oder Benzylrest und m für die Ziffern 1 bis 4 steht,

-(CH₂)ₘ(C₆H₄)-O-R⁶,

worin R⁶ für ein Wasserstoffatom, einen C₁-C₆-Alkylrest oder einen Phenyl- oder Benzylrest und m für die Ziffern 1 bis 4 steht sowie -CH₂(C₆H₄)-O-R⁷ steht, worin R⁷ für einen Methyl-, Ethyl-, n-Propyl-, iso-Propyl, n-Butyl-, iso-Butyl-, tert-Butyl-, Phenyl- oder Benzylrest steht.

Ganz besonders bevorzugte Gruppen Z¹ sind Reste der Formeln -CH₂-C₆H₄-OC₂H₅, -CH₂-C₆H₄-C₄H₉, -CH₂-C₆H₄-OC₄H₉, -CH₂-C₆H₄-CH₂C₆H₅ und -CH₂-C₆H₄-O-CH₂C₆H₅.

Als Gruppe Z² kommen alle als Z¹ genannten Reste in Frage.

Bevorzugt steht Z² für ein Wasserstoffatom, wenn Z¹ für die Reste -CH₂-C₆H₄-C₄H₉, -CH₂-C₆H₄-OC₄H₉, -CH₂-C₆H₄-CH₂C₆H₅ oder -CH₂-C₆H₄-O-CH₂C₆H₅ steht.

Z² steht nicht für ein Wasserstoffatom, wenn Z¹ für einen unsubstituierten C₆-C₁₀-Arylrest steht.

Die Gruppen Z¹ und Z² können auch zusammen unter Einbeziehung des Kohlenstoffatoms, dessen Substituenten sie sind, einen Ring oder einen Bizyklus bilden. Beispielhaft seien gezeigt:

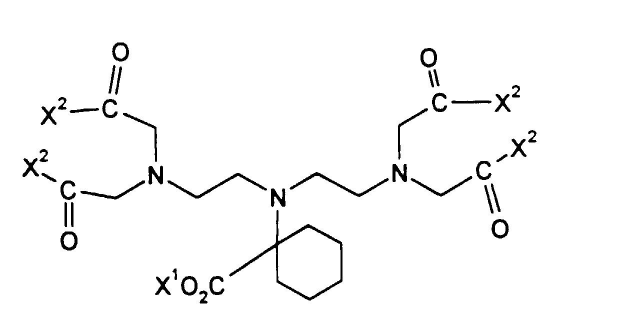

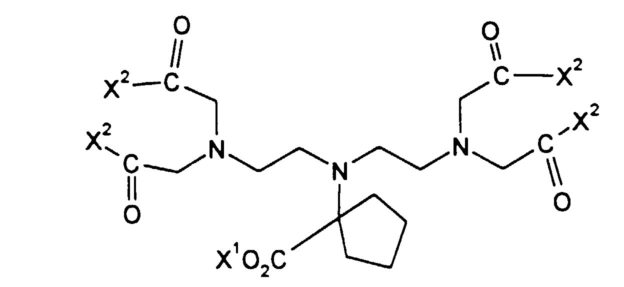

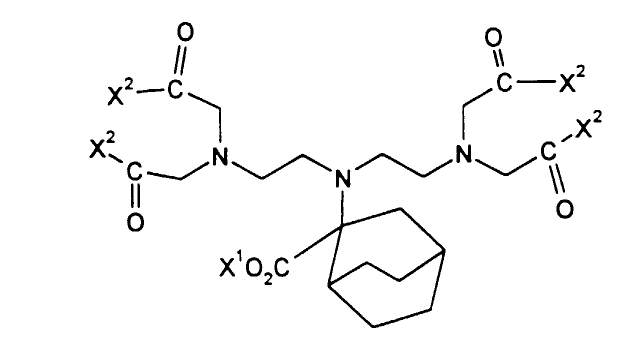

Als physiologisch verträgliche Kationen seien beispielhaft genannt Natrium⁺, Kalcium²⁺, Magnesium²⁺ und Zink²⁺ sowie organische Kationen wie Meglumin, Glucosamin, Arginin, Omithin, Lysin und Ethanolamin.

### Herstellung der erfindungsgemäßen Komplexe

Die Herstellung der erfindungsgemäßen Komplexe erfolgt in der Weise, wie sie in den Patentschriften EP 71564, EP 130934 und DE-OS 3401052 offenbart worden ist, indem man das Metalloxid oder ein Metallsalz (beispielsweise ein Chlorid, Nitrat, Acetat, Carbonat oder Sulfat) des Elements der Ordnungszahlen 20 - 32, 39 - 51 oder 57 - 83 in Wasser und/oder einem anderen polaren Lösungsmittel (wie Methanol, Ethanol, Isopropanol oder N,N-Dimethylformamid) löst oder suspendiert und mit der Lösung oder Suspension der äquivalenten Menge des Komplexbildners der allgemeinen Formel Ib 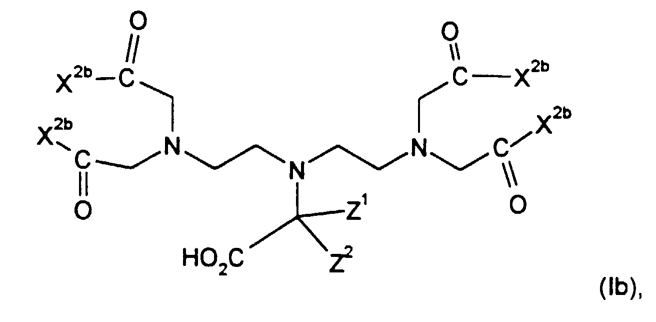 worin
- Z¹ und Z²: die oben genannten Bedeutungen haben,
- X^{2b}: unabhängig voneinander für eine Gruppe
HO- oder
N(R¹)R² mit R¹, R² in der oben genannten Bedeutung, stehen,
umsetzt und anschließend, falls gewünscht, vorhandene acide Wasserstoffatome von Säuregruppen durch Kationen anorganischer und/oder organischer Basen oder Aminosäuren substituiert.

Falls in den Gruppen Z¹, Z², R¹ und R² funktionelle Reste vorliegen, können diese bei der Komplexbildung in geschützter Form vorlegen. Die Abspaltung der Schutzgruppen kann dann in einen nachfolgenden Schritt vorgenommen werden.

Die Neutralisation erfolgt dabei mit Hilfe anorganischer Basen (z.B. Hydroxiden, Carbonaten, oder Bicarbonaten) von z.B. Natrium, Kalium oder Lithium und/oder organischer Basen wie unter anderem primärer, sekundärer und tertiärer Amine, wie z.B. Ethanolamin, Glucamin, N-Methyl- und N,N-Dimethylglucamin, sowie basischer Aminosäuren, wie z.B. Lysin, Arginin und Ornithin.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol etc.) niederen Ketonen (Aceton etc.) polaren Ethern (Tetrahydrofuran, Dioxan, 1,2-Dimethoxyethan etc.) auszufällen und so leicht zu isolierende und gut zu reinigende Kristallisate zu erhalten.

Enthalten die sauren Komplexe mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den Komplexbildner in wäßriger Suspension oder Lösung mit dem Oxid oder Salz des gewünschten Elementes und der Hälfte der zur Neutralisation benötigten Menge einer organischen Base umsetzt, das gebildete Komplexsalz isoliert, es gewünschtenfalls aufreinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen, wie z.B. in der EP 0450742 beschrieben, ganz oder teilweise in Amide zu überführen.

Sollen die erfindungsgemäßen Mittel Radioisotope enthalten, kann die Herstellung der Komplexe aus den Komplexbildnern nach den in "Radiotracers for Medical Applications", Vol. I, CRC Press, Boca Raton, Florida beschriebenen Methoden erfolgen.

### Herstellung der erfindungsgemäßen Komplexbildner

Die Herstellung von Verbindungen der allgemeinen Formel I, erfolgt beispielsweise dadurch, daß zunächst ein Aminosäurederivat der allgemeinen Formel II 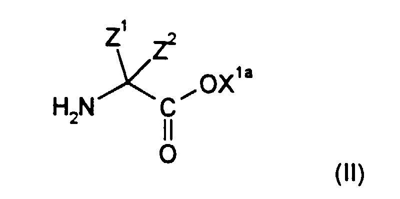 worin
- Z¹ und Z²: die oben angegebene Bedeutung besitzen und
- X^{1a}: für eine geradkettige oder verzweigte C₁-C₄-Alkylgruppe oder eine gegebenfalls substituierte Benzylgruppe, bevorzugt eine tert.-Butyl- oder eine Benzylgruppe steht,
mit zwei Alkylierungsbausteinen der allgemeinen Formel III umgesetzt wird 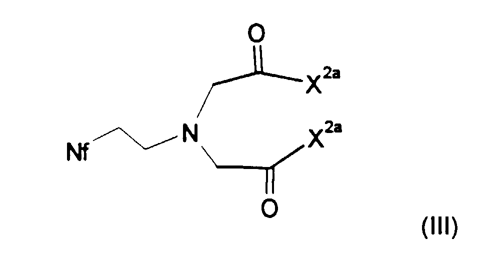 in denen
- Nf: für ein Nucleofug, wie z.B. ein Chlorid, Bromid, lodid, O-Mesylat, O-Tosylat oder O-Triflat und
- X^{2a}: jeweils unabhängig voneinander für eine Gruppe
X^{1a}-O mit X^{1a} in der in Formel II genannten Bedeutung oder
N(R¹)R² mit R¹ und R² in der oben genannten Bedeutung
steht.

Bevorzugte Aminosäurederivate der allgemeinen Formel II sind die Ester von kommerziell erhältlichen oder literaturbekannten α-Aminosäuren. Besonders bevorzugt sind die Ester von α-Aminosäuren die eine längere Alkylkette (>C₄) und/oder einen aromatischen Rest enthalten. Ganz besonders bevorzugt sind Ester von 3-Phenylalaninderivaten, deren Phenylrest in der 4-Position mit einem Alkyl- oder Alkoxyrest substituiert ist.

Die Reaktion von Verbindung II mit Verbindung III erfolgt bevorzugt in einer gepufferten Alkylierungsreaktion, wobei als Puffer eine wäßrige Phosphat-Pufferlösung dient. Die Umsetzung erfolgt bei pH-Werten von 7-11, bevorzugt jedoch bei pH 8. Die Pufferkonzentration kann zwischen 0,1 - 2,5 M liegen, bevorzugt wird jedoch eine 2 M-Phosphat-Pufferlösung verwendet. Die Temperatur der Alkylierung kann zwischen 0 und 50°C liegen, die bevorzugte Temperatur ist Raumtemperatur.

Die Reaktion wird in einem polaren Lösungsmittel, wie z.B. Acetonitril, Tetrahydrofuran, 1,4-Dioxan oder 1,2-Dimethoxyethan durchgeführt. Bevorzugt wird Acetonitril verwendet. Die Alkylierung des Amins der Formel II mit Verbindungen der Formel III kann aber auch in einem polaren, aprotischen Lösungsmittel unter Verwendung einer Hilfsbase (wie z.B. Triethylamin) erfolgen.

Die in der Reaktion eingesetzten α-Aminosäureester der allgemeinen Formel II können nach den dem Fachmann bekannten Methoden (z.B. Houben-Weyl, Methoden der organischen Chemie, Synthese von Peptiden, Teil II, Band XV/2, Georg Thieme Verlag Stuttgart, 1974, S. 3 ff) aus den im Handel erhältlichen Aminosäuren hergestellt werden. Als Kaufware sind α-Aminosäuren und -derivate z.B. bei der Fluka Chemie AG, CH-9470 Buchs oder der BACHEM Feinchemikalien AG, CH-4416 Bubendorf erhältlich.

Bevorzugte Aminosäurederivate der allgemeinen Formel II sind die Aminosäurebenzyl-, Aminosäure-tert.-butyl-, Aminosäureisopropyl- und Aminosäureethylester. Bei der Synthese dieser Verbindungen fallen in der Regel Salze (wie z.B. Hydrochloride, Hydrosulfate, Sulfate, Phosphate oder p-Toluolsulfonate) an, die direkt in die Reaktion eingesetzt werden können.

Der in die Alkylierung eingesetzte Baustein der allgemeinen Formel III kann für den Fall, daß X² für die tert.-Butoxygruppe und Nf für ein Bromatom steht analog der Beschreibung von Rapoport (J.Org.Chem., 58, 1151 (1993)) hergestellt werden. Für den Fall, daß eine oder beide Gruppen X² die Bedeutung eines Amides haben sollte, kann die Herstellung analog der in Beispiel 4 genannten Weise hergestellt werden oder nach anderen, dem Fachmann geläufigen Verfahren.

Nach der N,N-Dialkylierung des jeweiligen α-Aminosäureesters (z.B. des Tyrosinbenzylesters) können weitere Umsetzungen an dessen funktionellen Gruppen erfolgen (in diesem Falle z.B. eine O-Alkylierung des erhaltenen Tyrosinderivates).

Die Komplexbildner der allgemeinen Formel Ib werden durch Abspaltung der Säureschutzgruppen X^{1a} aus den erhaltenen Verbindungen der allgemeinen Formel la 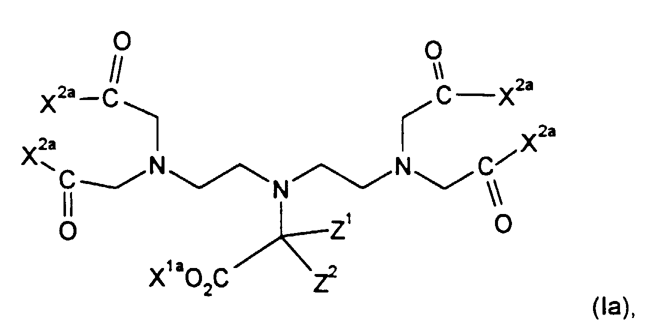 worin
- X^{1a}: für eine geradkettige oder verzweigte C₁-C₄-Alkylgruppe oder eine Benzylgruppe, bevorzugt eine tert.-Butyl-, Ethyl- Isopropyl- oder eine - gegebenenfalls substituierte - Benzylgruppe steht und
- X^{2a}: jeweils unabhängig voneinander für eine Gruppe
O-X^{1a} mit X^{1a} in der oben genannten Bedeutung oder
N(R¹)R² mit R¹ und R² in der oben genannten Bedeutung
steht, hergestellt.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wäßriger oder wäßrig-alkoholischer Lösung bei Temperaturen von 0° bis 80°C, saure Verseifung mit Mineralsäuren oder im Fall von ^{*tert*}^{.-}Butylestern mit Hilfe von Trifluoressigsäure.

Enthalten die Gruppen Z¹, Z², R¹ und R² reaktive funktionelle Gruppen, kann die Einführung von Schutzgruppen nötig sein. In diesem Falle liegen diese Reste bereits in den Verbindungen der allgemeinen Formeln II und III in geschützter Form vor. Die Abspaltung der in den Gruppen Z¹, Z², R¹ und R² gegebenenfalls enthaltenen Schutzgruppen kann - insbesondere wenn es sich dabei um Säureschutzgruppen handelt - gemeinsam mit der Entfernung der Schutzgruppen X^{1a} erfolgen. Die Abspaltung der in den Gruppen Z¹, Z², R¹ und R² gegebenenfalls enthaltenen Schutzgruppen kann aber auch separat von der Entfernung der Schutzgruppen X^{1a} vorgenommen werden.

Die Erfindung betrifft daher auch die Verfahren zur Herstellung der erfindungsgemäßen Komplexe und Komplexbildner.

### Pharmazeutische Mittel

Ein weiterer Gegenstand der Erfindung sind Mittel, welche mindestens eine der erfindungsgemäßen Verbindungen enthalten sowie ein Verfahren zur Herstellung dieser Mittel, welches dadurch gekennzeichnet ist, daß das in Wasser gelöste Komplexsalz mit den in der Galenik üblichen Zusätzen und Stabilisatoren in eine für die enterale bzw. parenterale Applikation geeignete Form gebracht wird, so daß das Komplexsalz in einer Konzentration von 1 bis 1500 mmol/l vorzugsweise in einer Konzentration von 10 bis 1000 mmol/l vorliegt. Die resultierenden Mittel werden anschließend gewünschtenfalls sterilisiert. Sie werden in Abhängigkeit von der diagnostischen Fragestellung in der Regel in einer Dosis von 1 bis 300 ml appliziert.

Geeignete Zusätze sind beispielsweise physiologisch unbedenkliche Puffer (wie z. B. Tromethamin), geringe Zusätze von Komplexbildnern (wie z. B. Diethylentriaminpentaessigsäure oder einem erfindungsgemäßen Komplexbildner der allgemeinen Formel I), geringe Zusätze von Calciumsalzen oder -komplexen dieser Komplexbildnern oder, falls erforderlich, Elektrolyte wie z. B. Natriumchlorid oder, falls erforderlich, Antioxidantien wie z. B. Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoffen (z. B. Methylcellulose, Lactose, Mannit) und/oder Tensiden (z.B. Lecithine, Tweens®, Myrj® und/oder Aromastoffen zur Geschmackskorrektur (z. B. ätherischen Ölen) gemischt.

Prinzipiell ist es auch möglich, die erfindungsgemäßen diagnostischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen Substanzen erfüllen die vielfältigen Voraussetzungen, die an Kontrastmittel in der modernen Diagnostik zu stellen sind. Die Verbindungen und aus ihnen hergestellte Mittel zeichnen sich aus durch:
- einen hohen Absorptionskoeffizienten für Röntgenstrahlen,
- eine Relaxivität,
- eine gute Verträglichkeit, die notwendig ist, um den nichtinvasiven Charakter der Untersuchungen aufrechtzuerhalten,
- eine hohe Wirksamkeit, die notwendig ist, um den Körper mit möglichst geringen Mengen an Fremdstoffen zu belasten,
- eine gute Wasserlöslichkeit (Diese erlaubt es, hochkonzentrierte Lösungen herzustellen, wie sie besonders für die Verwendung als Röntgenkontrastmittel erforderlich sind. Damit kann die Volumenbelastung des Kreislaufs in vertretbaren Grenzen gehalten werden.),
- eine geringe Viskosität,
- geringe Osmolalität,
- günstige Ausscheidungskinetik.

Weiterhin weisen die erfindungsgemäßen Mittel nicht nur eine hohe Stabilität in vitro auf, sondern auch eine überraschend hohe Stabilität in vivo, so daß eine Freigabe oder ein Austausch der in den Komplexen nicht kovalent gebundenen - an sich giftigen - lonen innerhalb der Zeit, in der die neuen Kontrastmittel vollständig wieder ausgeschieden werden, nicht erfolgt.

Neben der hohen Wasserlöslichkeit, welche überraschenderweise bei Anwesenheit von Metallionen in einen für die Röntgendiagnostik erforderlichen Bereich gesteigert werden konnte, wirken sich die erfindungsgemäße Komplexverbindungen in der Röntgendiagnostik dadurch positiv aus, daß sie Untersuchungen bei kurzwelligerer Röntgenstrahlung gestatten als dies mit konventionellen Kontrastmitteln möglich ist, wodurch die Strahlenbelastung des Patienten deutlich gemindert wird, da bekanntermaßen weiche Strahlung vom Gewebe sehr viel stärker absorbiert wird als harte (R. Felix, Das Röntgenbild; Thieme Stuttgart 1980).

Für die Anwendung in der Röntgendiagnostik sind die erfindungsgemäßen Komplexe folgender Metalle besonders geeignet: Gadolinium, Terbium, Dysprosium, Holmium, Erbium, Ytterbium, Lutetium, Wismut und Hafnium.

Wegen der günstigen Absorptionseigenschaften der erfindungsgemäßen Kontrastmittel im Bereich harter Röntgenstrahlung, sind die Mittel auch besonders für digitale Substraktionstechniken (die mit höheren Röhrenspannungen arbeiten) geeignet.

Hervorzuheben ist weiterhin, daß sich die erfindungsgemäßen Verbindungen im Vergleich mit anderen Komplexverbindungen durch eine verbesserte Herz-/Kreislaufverträglichkeit auszeichnet.

Besonders hervorzuheben ist das überraschend günstige in vivo Verteilungsverhalten der erfindungsgemäßen Mittel. Dieses gestattet erstmalig, mit einer für Röntgenkontrastmittel niedrigen Dosis (0,1 - 1 mmol/kg Körpergewicht ), Röntgenaufnahmen von hohem diagnostischen Aussagewert im Bereich der Leber, sowie der Gallengänge und der Galle anzufertigen.

Die erfindungsgemäßen Mittel, die im Komplex ein paramagnetisches Metallion eines Elementes der Ordnungszahlen 20 - 32, 39 - 51 oder 57 - 83 enthalten, können neben der Verwendung in der Röntgendiagnostik auch in der NMR-Diagnostik eingesetzt werden. Dieser duale Charakter eröffnet weitere Einsatzgebiete. So sind diese erfindungsgemäßen Mittel immer dann mit Vorteil anzuwenden, wenn zur differenzierten Darstellung und zuverlässigen Bestimmung bestimmter Erkrankungen eine Kombination der Röntgen- und NMR-Diagnostik erforderlich ist. Dies trifft z.B. zu bei Rezidivverdacht nach Tumoroperationen oder Bestrahlungstherapien. In diesen Fällen wird dem Patienten durch Verwendung eines Kontrastmittels, das für beide Techniken gleichermaßen geeignet ist, eine zusätzlich Belastung durch doppelte Applikation erspart.

Die erfindungsgemäßen Komplexbildner und deren Komplexe mit schwach gebundenen Metallen sind darüber hinaus dazu geeignet, Schwermetalle aus dem Körper zu entfernen, zum Beispiel nach einer Schwermetallvergiftung. Durch die extrarenale Ausscheidung der erfindungsgemäßen Komplexbildner und Komplexe ist insbesondere eine Entgiftung der Leber möglich. Gegenstand der Erfindung sind daher auch die Verwendung der erfindungsgemäßen Verbindungen zur Herstellung von Mitteln zur Behandlung von Schwermetallvergiftungen, insbesondere zur Behandlung von Schwermetallvergiftungen der Leber.

Weitere Gegenstände der Erfindung sind durch die Ansprüche gekennzeichnet.

Insgesamt ist es gelungen, mit den genannten Komplexverbindungen neue Möglichkeiten in der diagnostischen und therapeutischen Medizin zu erschließen.

### Beispiele

Die nachfolgenden Beispiele dienen zur näheren Erläuterung des Erfindungsgegenstandes ohne ihn auf diese beschränken zu wollen.

### Beispiel 1

### Dysprosiumkomplex des Dinatriumsalzes von N,N-Bis-{2-[N',N'-bis-(carboxymethyl)]-amino]-ethyl}-L-3-[(4-ethoxy)-phenyl]-alanin

### a) N,N-Bis-{2-[N',N'-bis-[(benzyloxycarbonyl)-methyl]-amino]-ethyl}-L-tyrosinbenzylester

15,5 g (35,0 mmol) L-Tyrosinbenzylester-4-methylbenzolsulfonat und 33,2 g (79,0 mmol) N,N-Bis-[(benzyloxycarbonyl)-methyl]-2-bromethylamin (M. Williams und H. Rapoport, J. Org. Chem. 58, 1151 (1993)) werden in 70 ml Acetonitril vorgelegt und mit 60 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.

Ausbeute: 23,4 g (70,3 % d. Th.) farbloses Öl.

| Analyse (bezogen auf lösungsmittelfreie Substanz): | | | | |
|---|---|---|---|---|
| ber. | C 70,79 | H 6,26 | N 4,42 | O 18,52 |
| gef. | C 70,69 | H 6,33 | N 4,51 | |

### b) N,N-Bis-{2-[N',N'-bis-[(benzyloxycarbonyl)-methyl]-amino]-ethyl}-L-3-[(4-ethoxy)-phenyl]-alaninbenzylester

20,3 g (21,4 mmol) der nach Beispiel 1a) herstellten Verbindung werden in 50 ml wasserfreiem N,N-Dimethylformamid gelöst und bei 0°C unter Argon mit 0,94 g(23,5 mmol) Natriumhydriddispersion (60 % in Mineralöl) versetzt. Man läßt den Ansatz 15 Minuten rühren, gibt dann 3,74 g (24,0 mmol) Ethyliodid zu, läßt die Reaktionstemperatur auf Raumtemperatur steigen und rührt weitere 5 Stunden. Zur Aufarbeitung wird der Ansatz in Toluol aufgenommen und mehrmals gegen wässrige Natriumhydrogencarbonatlösung ausgeschüttelt. Die organische Phase wird abgetrennt, über Magnesiumsulfat getrocknet, filtriert und eingedampft. Der ölige Rückstand wird an Kieselgel mit Hexan/Diethylether/Triethylamin (30:80:1) chromatographiert, die produkthaltigen Fraktionen werden vereint und eingedampft.

Ausbeute: 18,8 g (89,8 % d. Th.) farbloses Öl

| Analyse (bezogen auf lösungsmittelfreie Substanz): | | | | |
|---|---|---|---|---|
| ber. | C 71,22 | H 6,49 | N 4,30 | O 17,99 |
| gef. | C 71,23 | H 6,48 | N 4,37 | |

### c) N,N-Bis-{2-[N',N'-bis-(carboxymethyl)]-amino]-ethyl}-L-3-[(4-ethoxy)-phenyl]-alanin

16,8 g (17,2 mmol) des in Beispiel 1b) beschriebenen Decaesters werden in 145 ml Methanol gelöst, mit 68,8 ml 2n Natronlauge versetzt und ca 5 Stunden bei 60°C gerührt. Anschließend wird die Lösung mit konzentrierter Salzsäure auf pH 1 eingestellt und eingedampft; der Rückstand wird auf eine Säule mit stark saurem lonentauscher gegeben und mit wässriger Ammoniaklösung eluiert. Die das Produkt enthaltenden Eluatfraktionen werden im Vakuum eingedampft und am Ölpumpenvakuum getrocknet.

Ausbeute: 9,01 g (99,4.% d Th.)

| Analyse (bezogen auf lösungsmittelfreie Substanz): | | | | |
|---|---|---|---|---|
| ber. | C 52,37 | H 6,31 | N 7,97 | O 33,36 |
| gef. | C 52,21 | H 6,32 | N 7,87 | |

### d) Dysprosiumkomplex des Dinatriumsalzes von N,N-Bis-{2-[N',N'-bis-(carboxymethyl)]-amino]-ethyl}-L-3-[(4-ethoxy)-phenyl]-alanin

8,45 g (16,02 mmol) der in Beispiel 1c) beschriebenen Decasäure werden in 25 ml Wasser aufgenommen, mit 2,57 g (7,10 mmol) Dysprosiumoxid versetzt und 3 Stunden bei 60°C gerührt. Anschließend stellt man mit verdünnter Natronlauge pH 7,2 ein, filtriert und gefriertrocknet das Filtrat.

Ausbeute: 11,54 g (98.6 % d. Th.) farbloses Lyophilisat

| Analyse (bezogen auf wasserfreie Substanz): | | | | | |
|---|---|---|---|---|---|
| ber. | C 37,79 | H 3,86 | Dy 20,89 | N 5,58Na 6,11 | O 23,38 |
| gef. | C 37,88 | H 3,91 | Dy 20,80 | N 5,62Na 6,18 | |

Folgende Komplexe werden in analoger Weise hergestellt:

### a) Wismutkomplex des Dinatriumsalzes von N,N-Bis-{2-[N',N'-bis-(carboxymethyl)]-amino]-ethyl}-L-3-[(4-ethoxy)-phenyl]-alanin

| Analyse (bezogen auf wasserfreie Substanz): | | | | | |
|---|---|---|---|---|---|
| ber. | C 35,53 | H 3,63 | Bi 26,88 | N 5,41 Na 5,91 | O 22,64 |
| gef. | C 35,24 | H 3,82 | Bi 26,76 | N 5,33Na 5,63 | |

### b) Mangankomplex des Trinatriumsalzes von N,N-Bis-{2-[N',N'-bis-(carboxymethyl)]-amino]-ethyl}-L-3-[(4-ethoxy)-phenyl]-alanin

| Analyse (bezogen auf wasserfreie Substanz): | | | | | |
|---|---|---|---|---|---|
| ber. | C 42,74 | H 4,37 | Mn 8,50 | N 6,50Na 10,67 | O 27,23 |
| gef. | C 42,58 | H 4,51 | Mn 8,36 | N 6,47Na 10,54 | |

### Beispiel 2

### Gadoliniumkomplex des Dinatriumsalzes von N,N-Bis-[2-[N',N'-bis-(carboxymethyl)-amino]-ethyl]-α-aminolaurinsäure

### a) α-Aminolaurinsäureisopropylester

50 ml Isopropanol werden bei 0°C unter Argon gerührt und tropfenweise mit 3,12 ml (41,6 mmol) Thionylchlorid versetzt. 30 Minuten später gibt man 7,40 g (34,4 mmol) α-Aminolaurinsäure portionsweise zu, rührt eine Stunde bei Raumtemperatur und läßt den Ansatz anschließend zwei Stunden unter Rückfluß kochen. Nach dem Abkühlen auf Raumtemperatur dampft man den Ansatz vollständig ein, nimmt den Rückstand in tert.-Butylmethylether auf und schüttelt gegen wässrige Natriumcarbonatlösung aus. Die organische Phase wird über Magnesiumsulfat getrocknet, filtriert und eingedampft.

Ausbeute: 7,49 g (92,1 % d. Th.) farbloses Öl.

| Analyse: | | | | |
|---|---|---|---|---|
| ber. | C 69,09 | H 12,01 | N 5,75 | O 13,15 |
| gef. | C 69,02 | H 12,22 | N 5,81 | |

### b) N,N-Bis-[2-[N',N'-bis-[(tert.-butyloxycarbonyl)-methyl]-amino]-ethyl]-α-aminolaurinsäureisopropylester

7,95 g (32,7 mmol) des entsprechend Beispiel 2a) hergestellten Amins und 25,4 g (72,0 mmol) N,N-Bis-[(tert.-butyloxycarbonyl)-methyl]-2-bromethylamin (M. Williams und H. Rapoport, J. Org. Chem. 58, 1151 (1993)) werden in 50 ml Acetonitril vorgelegt und mit 20 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.

Ausbeute: 16,0 g (62,3 % d. Th.) gelbliches Öl.

| Analyse (bezogen auf lösungsmittelfreie Substanz): | | | | |
|---|---|---|---|---|
| ber. | C 64,17 | H 10,13 | N 5,35 | O 20,35 |
| gef. | C 64,20 | H 10,24 | N 5,43 | |

### c) N, N-Bis-[2-[N',N'-bis-(carboxymethyl)-amino]-ethyl]-α-aminolaurinsäure

15,3 g (19,5 mmol) des in Beispiel 2b) beschriebenen tert.-Butylesters werden in 70 ml Methanol gelöst, mit 77,9 ml 2n Natronlauge versetzt und ca. 5 Stunden bei 60°C gerührt. Anschließend wird die Lösung mit konzentrierter Salzsäure auf pH 1 eingestellt und eingedampft; der Rückstand wird auf eine Säule mit stark saurem lonenaustauscher gegeben und mit wässriger Ammoniaklösung eluiert. Die das Produkt enthaltenden Eluatfraktionen werden im Vakuum eingedampft und am Ölpumpenvakuum getrocknet.

Ausbeute: 9,01 g (86,8 % d. Th.) hellbeiger Feststoff

| Analyse (bezogen auf wasserfreie Substanz): | | | | |
|---|---|---|---|---|
| ber. | C 54,02 | H 8,12 | N 7,88 | O 29,98 |
| gef. | C 54,11 | H 8,05 | N 7,79 | |

### d) Gadoliniumkomplex des Dinatriumsalzes von N,N-Bis-[2-[N',N'-bis-(carboxymethyl)-amino]-ethyl]-α-aminolaurinsäure

Eine Suspension von 8,43 g (15,7 mmol) der nach Beispiel 2c) hergestellten Pentasäure in 100 ml Wasser wird mit 2,86 g (7,90 mmol) Gadoliniumoxid versetzt und bei 80°C 2 Stunden gerührt. Dann werden mit einer Mikrobürette 31,6 ml einnormale Natronlauge zugegeben und 1 Stunde nachgerührt. Anschließend wird die Lösung bei 80°C nach Zugabe von 0,5 g Aktivkohle 2 Stunden gerührt und filtriert. Das Filtrat ergibt nach Gefriertrocknen einen farblosen Feststoff.

Ausbeute 10,73 g (92,8 % d. Th.)

| Analyse (bezogen auf wasserfreie Substanz): | | | | | | |
|---|---|---|---|---|---|---|
| ber. | C 39,39 | H 5,23 | N 5,74 | O 19,81 | Gd 21,49 | Na 6,28 |
| gef. | C 39,44 | H 5,11 | N 5,80 | | Gd 21,39 | Na 6,30 |

### Beispiel 3

### Ytterbiumkomplex des Dinatriumsalzes von N_{α},N_{α}-Bis-[2-[N',N'-bis-(carboxymethyl)-amino]-ethyl]-N_{ε}-benzoyl-L-lysin

### a) N_{ε}-Carboxybenzyl-L-lysinisopropylester, Hydrochlorid

28,0 g (100 mmol) N_{ε}-Carboxybenzyl-L-lysin werden in eine bei 0°C gerührte Lösung von 13,0 g (110 mmol) Thionylchlorid in 150 ml Isopropanol eingetragen. Anschließend läßt man eine Stunde bei Raumtemperatur weiterrühren und kocht dann eine Stunde unter Rückfluß. Beim Abkühlen fällt ein farbloser Niederschlag aus, der abgesaugt und im Vakuum getrocknet wird.

Ausbeute: 33,1 g (92,3 % d. Th.)

| Analyse: | | | | | |
|---|---|---|---|---|---|
| ber. | C 56,90 | H 7,58 | N 7,81 | 0 17,83 | Cl 9,88 |
| gef. | C 56,83 | H 7,44 | N 7,77 | | Cl 10,02 |

### b) N_{α},N_{α}-Bis-[2-[N',N'-bis-[(tert.-butyloxycarbonyl)-methyl]-amino]-ethyl]-N_{ε}-carboxybenzyl-L-lysinisopropylester

11,73 g (32,7 mmol) des entsprechend Beispiel 3a) hergestellten Hydrochlorids und 25,4 g (72,0 mmol) N,N-Bis-[(tert.-butyloxycarbonyl)-methyl]-2-bromethylamin (M. Williams und H. Rapoport, J. Org. Chem. 58, 1151 (1993)) werden in 50 ml Acetonitril vorgelegt und mit 20 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.

Ausbeute: 18,52 g (65,5 % d. Th.) gelbliches Öl.

| Analyse (bezogen auf lösungsmittelfreie Substanz): | | | | |
|---|---|---|---|---|
| ber. | C 62,48 | H 8,86 | N 6,48 | O 22,19 |
| gef. | C 62,37 | H 8,75 | N 6,53 | |

### c) N_{α} ,N_{α}-Bis-[2-[N',N'-bis-[(tert.-butyloxycarbonyl)-methyl]-amino]-ethyl]-L-lysinisopropylester

18,0 g (20.8 mmol) der in Beispiel 3b) beschriebenen Verbindung wurden in 200 ml Ethanol gelöst und nach Zugabe von 0.9 g Palladium auf Aktivkohle (10% Palladium) bis zur beendeten Wasserstoffaufnahme hydriert. Anschließend wurde filtriert und das Filtrat vollständig eingedampft.

Ausbeute: 15,2 g (100 % d. Th.) gelbliches Öl

| Analyse: | | | | |
|---|---|---|---|---|
| ber. | C 60.80 | H 9,65 | N 7,66 | O 21,89 |
| gef. | C 60,88 | H 9,75 | N 7,56 | |

### d) N_{α},N_{α}-Bis-[2-[N',N'-bis-[(tert.-butyloxycarbonyl)-methyl]-amino]-ethyl]-N_{ε}-benzoyl-L-lysinisopropylester

15,0 g (20,5 mmol) der nach Beispiel 3c) bereiteten Verbindung werden in 50 ml N,N-Dimethylformamid gelöst und unter Rühren bei 0°C tropfenweise mit 3.17 g (22,6 mmol) Benzoylchlorid versetzt. Anschließend wird über Nacht bei Raumtemperatur gerührt, im Vakuum eingedampft und der Rückstand mit Dichlormethan/Wasser ausgeschüttelt. Die organische Phase wird über Magnesiumsulfat getrocknet, filtriert, eingedampft und der Rückstand an Kieselgel mit Dichlormethan/Methanol (95:5) chromatographiert; die produkthaltigen Fraktionen ergeben nach Eindampfen ein farbloses Öl.

Ausbeute: 14,8 g (86,6 % d. Th.)

| Analyse: | | | | |
|---|---|---|---|---|
| ber. | C 63,29 | H 8,93 | N 6,71 | O 21,07 |
| gef. | C 63,18 | H 9;02 | N 6,66 | |

### e) N_{α} ,N_{α}-Bis-[2-[N',N'-bis-(carboxymethyl)-amino]-ethyl]-N_{ε}-benzoyl-L-lysin

Eine Suspension von 14,0 g (16,8 mmol) der nach Beispiel 3d) hergestellten Verbindung wird in 100 ml Methanol gelöst und mit 134 ml einnormaler Natronlauge versetzt. Man rührt ca. 5 Stunden bei 60°C und fällt die Pentasäure durch Zugabe von konzentrierter Salzsäure aus. Der farblose Niederschlag wurde abgesaugt und im Vakuum getrocknet. Das Rohprodukt wurde ohne weitere Reinigung für die weitere Stufe verwendet.

Ausbeute: 9.55 g (100 % d. Th.)

### f) Ytterbiumkomplex des Dinatriumsalzes von N_{α},N_{α}-Bis-[2-[N',N'-bis-(carboxymethyl)-amino]-ethyl]-N_{ε}-benzoyl-L-lysin

9,00 g (15,8 mmol) der nach Beispiel 3e) bereiteten Pentasäure werden in 100 ml Wasser mit 3,12 g (7,91 mmol) Ytterbiumoxid versetzt und bei 100°C 2 Stunden gerührt. Dann werden mit einer Mikrobürette 31,6 ml einnormale Natronlauge zugegeben und 1 Stunde nachgerührt. Anschließend wird die Lösung bei 80°C nach Zugabe von 0,5 g Aktivkohle 2 Stunden gerührt und filtriert. Das Filtrat ergibt nach Gefriertrocknen einen farblosen Feststoff.

Ausbeute 11,26 g (90,9 % d. Th.)

| Analyse (bezogen auf wasserfreie Substanz): | | | | | | |
|---|---|---|---|---|---|---|
| ber. | C 38,37 | H 3,99 | N 7,16 | O 22,49 | Yb 22,11 | Na 5,88 |
| gef. | C 38,44 | H 3,87 | N 7,20 | | Yb 22.07 | Na 5,91 |

### Beispiel 4

### Terbiumkomplex des Mononatriumsalzes der N, N-Bis-{2-[N'-(carboxymethyl)]-N'-[(benzylcarbamoyl)-methyl]-amino]-ethyl}-L-glutaminsäure

### a) N,N-Bis-{2-[N',N'-bis-((benzyloxycarbonyl)-methyl)-amino]-ethyl}-L-glutaminsäurediethylester

17,8 g (74,2 mmol) L-Glutaminsäurediethylester und 70,2 g (167 mmol) N,N-Bis-[(benzyloxycarbonyl)-methyl]-2-bromethylamin (M. Williams und H. Rapoport, J. Org. Chem. 58, 1151 (1993)) werden in 70 ml Acetonitril vorgelegt und mit 60 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.

Ausbeute: 46,0 g (70,3 % d. Th.) farbloses Öl.

| Analyse (bezogen auf lösungsmittelfreie Substanz): | | | | |
|---|---|---|---|---|
| ber. | C 66,73 | H 6,74 | N 4,76 | O 21,77 |
| gef. | C 66,69 | H 6,75 | N 4,81 | |

### b) N, N-Bis{2-[N',N'-bis-(carboxymethyl)-amino]-ethyl}-L-glutaminsäurediethylester

45,5 g (51,6 mmol) der nach Beispiel 4a) hergestellten Verbindung werden in 250 ml Ethanol gelöst und nach Zugabe von 2,5 g Palladium auf Aktivkohle (10 % Pd) unter Wasserstoffatmosphäre bis zur beendeten Wasserstoffaufnahme hydriert. Nach Filtrieren und Eindampfen des Filtrats im Vakuum erhält man einen farblosen Feststoff.

Ausbeute: 26,9 g (100 % d. Th.)

| Analyse: | | | | |
|---|---|---|---|---|
| ber. | C 48,36 | H 6,76 | N 8,06 | O 36,81 |
| gef. | C 48,33 | H 6,79 | N 8,10 | O 36,70 |

### c) N,N-Bis-[2-(2,6-dioxomorpholino)-ethyl]-L-glutaminsäurediethylester

26,0 g (49,9 mmol) der nach Beispiel 4b) hergestellten Verbindung werden in 100 ml Essigsäureanhydrid eine Stunde unter Rückfluß gekocht. Dann engt man bei Normaldruck auf ein Volumen von 50 ml ein und dampft im Vakuum vollständig ein. Der Rückstand wird in Toluol aufgenommen und die Lösung wird wieder eingedampft; dieser Vorgang wird dreimal wiederholt. Zuletzt wird der Rückstand am Ölpumpenvakuum sorgfältig getrocknet.

Ausbeute: 22,9 g (100 % d. Th.) farbloses Öl

| Analyse | | | | |
|---|---|---|---|---|
| ber. | C 51,95 | H 6,44 | N 8,66 | O 32,96 |
| gef. | C 51,81 | H 6,60 | N 8,77 | |

### d) Terbiumkomplex des Mononatriumsalzes der N,N-Bis-{2-[N'-(carboxymethyl)]-N'-[(benzylcarbamoyl)-methyl]-amino]-ethyl}-L-glutaminsäure

22,0 g (45,3 mmol) der nach Beispiel 4c) hergestellten Verbindung werden in 50 ml Tetrahydrofuran gelöst und nach Zugabe von 9,71 g (90,6 mmol) Benzylamin bis zur (mittels Dünnschichtchromatographie festgestellten) vollständigen Umsetzung bei 50°C gerührt. Dann gibt man 102 ml zweinormale Natronlauge zu, rührt 2 Stunden bei 60°C, stellt mit konzentrierter Salzsäure pH 1 ein, engt am Rotationsverdampfer stark ein und reinigt den Rückstand durch lonenaustauschchromatographie (Kationenaustauscher (H⁺-Form), Eluens: wässrige Ammoniaklösung). Das Eluat wird eingedampft und am Hochvakuum scharf getrocknet, wodurch der freie Komplexligand erhalten wird.

Die Hexasäure wird in 250 ml Wasser aufgenommen und mit 8,29 g (22,7 mmol) Terbiumoxid versetzt. Die Suspension wird 2 Stunden bei 90°C gerührt und filtriert. Dann wird mit einnormaler Natronlauge pH 7,3 eingestellt. Anschließend wird die Lösung bei 80°C nach Zugabe von 0,5 g Aktivkohle 2 Stunden gerührt und filtriert. Das Filtrat ergibt nach Gefriertrocknen einen farblosen Feststoff.

Ausbeute 27,4 g (73,6 % d. Th.)

| Analyse (bezogen auf wasserfreie Substanz): | | | | | | |
|---|---|---|---|---|---|---|
| ber. | C 45,32 | H 4,54 | N 8,52 | O 19,47 | Tb 19,34 | Na 2,80 |
| gef. | C 45,45 | H 4,68 | N 8,57 | | Tb 19,27 | Na 2,77 |

### Beispiel 5

### Ytterbiumkomplex des Trinatriumsalzes von N,N-Bis-{2-[N',N'-bis-(carboxymethyl)]-amino]-ethyl}-3-[4-(carboxypropionylamino)-phenyl]-alanin

### a) N,N-Bis-{2-[N',N'-bis-(carboxymethyl)]-amino]-ethyl}-3-[4-(carboxypropionylamino)-phenyl]-alanin

Zu einer unter Feuchtigkeitsausschluß gerührten Suspension von 24,9 g (50 mmol) N,N-Bis-{2-[N',N'-bis-(carboxymethyl)]-amino]-ethyl}-3-[4-aminophenyl]-alanin (M. Williams und H. Rapoport, J. Org. Chem. 58, 1151 (1993)) in 200 ml wasserfreiem Dioxan werden 5,2 g (52 mmol) Bernsteinsäureanhydrid bei Raumtemperatur zugegeben. Der Ansatz wird sechs Stunden bei Raumtemperatur gerührt und anschließend eingedampft. Man nimmt den Rückstand in halbnormaler Natronlauge auf, extrahiert mit Essigsäureethylester und fällt die Säure mit konzentrierter Salzsäure bei pH 1 aus der wäßrigen Phase.

Ausbeute: 25,2 g (84,2 % d. Th.) farbloser Feststoff.

| Analyse (bezogen auf lösungsmittelfreie Substanz): | | | | |
|---|---|---|---|---|
| ber. | C 50,17 | H 5,72 | N 9,36 | O 34,75 |
| gef. | C 50,26 | H 5,64 | N 9,18 | |

### b) Ytterbiumkomplex des Trinatriumsalzes von N,N-Bis-{2-[N',N'-bis-(carboxymethyl)]-amino]-ethyl}-3-[4-(carboxypropionylamino)-phenyl]-alanin

Eine Suspension von 23,94 g (40 mmol) N,N-Bis-{2-[N',N'-bis-(carboxymethyl)]-amino]-ethyl}-3-[4-(carboxypropionylamino)-phenyl]-alanin in 285 ml Wasser wird mit 7,88 g (20 mmol) Ytterbiumoxid versetzt und bei 90°C vier Tage gerührt. Dann werden mit einer Mikrobürette 80,5 ml einnormale Natronlauge zugegeben und zwei Stunden nachgerührt. Anschließend wird die Lösung bei 80°C nach Zugabe von 1,2 g Aktivkohle eine Stunde gerührt und filtriert. Das Filtrat ergibt nach der Gefriertrocknung einen farblosen Feststoff.

Ausbeute 28,7 g (86 % d. Th.)

| Analyse (bezogen auf wasserfreie Substanz): | | | | | | |
|---|---|---|---|---|---|---|
| ber. | C 35,98 | H 3,38 | N 6,71 | O 24,92 | Yb 20,74 | Na 8,26 |
| gef. | C 35,75 | H 3,44 | N 6,58 | | Yb 20,63 | Na 8,01 |

Folgender Komplex wird in analoger Weise hergestellt:

Hafniumkomplex des Dinatriumsalzes von N,N-Bis-{2-[N',N'-bis-(carboxymethyl)]-amino]-ethyl}-3-[4-(carboxypropionylamino)-phenyl]-alanin

| Analyse (bezogen auf wasserfreie Substanz): | | | | | | |
|---|---|---|---|---|---|---|
| ber. | C 36,75 | H 3,45 | N 6,86 | O 25,46 | Hf 21,85 | Na 5,63 |
| gef. | C 36,66 | H 3,61 | N 6,70 | | Hf 21,67 | Na 5,35 |

### Beispiel 6

### Hafniumkomplex des Dinatriumsalzes des N,N-Bis-{2-[N',N'-bis-(carboxymethyl)-amino]-ethyl}-2-(10-carboxydecyl)-glycin

### a) 11 -Bromundecansäure-iso-propyl-ester

59 g (222 mmol) 11-Bromundecansäure werden in 86 ml (1,11 mol) Isopropanol suspendiert und mit 13 ml konzentrierter Schwefelsäure versetzt. Man refluxiert die Reaktionsmischung über Nacht, stellt anschließend mit Natronlauge auf pH 8,5 und extrahiert mit Essigsäureethylester. Die organische Phase wird mit Wasser gewaschen, über Natriumsulfat getrocknet, filtriert und eingedampft. Das Rohprodukt wird im Vakuum destilliert (bp 132-136°C).

Ausbeute: 53,1 g (77,8 % d. Th.) farbloses Öl.

| Analyse (bezogen auf lösungsmittelfreie Substanz): | | | | |
|---|---|---|---|---|
| ber. | C 54,73 | H 8,86 | O 10,41 | Br 26,00 |
| gef. | C 54,90 | H 8,58 | | Br 25,94 |

### b) N-(Diphenylmethylen)-2-(10-isopropoxycarbonyldecyl)-glycinethylester

13,37 g (50 mmol) N-(Diphenylmethylen)-glycinethylester werden in 100 ml Acetonitril gelöst, mit 1,6 g (5 mmol) Tetrabutylammoniumbromid, 20,7 g (150 mmol) Kaliumcarbonat und 15,4 g (50 mmol) 11 -Bromundecansäure-iso-propyl-ester versetzt. Die Suspension wird über Nacht am Rückfluß gekocht und nach beendeter Reaktion filtriert. Das Filtrat wird eingedampft, in Essigsäureethylester aufgenommen und die organische Phase mit Wasser gewaschen. nach dem Trocknen über Natriumsulfat wird filtriert und eingedampft.

Ausbeute: 21,2 g (85,9 % d. Th.) gelbliches Öl.

| Analyse (bezogen auf lösungsmittelfreie Substanz): | | | | |
|---|---|---|---|---|
| ber. | C 75,42 | H 8,78 | N 2,84 | O 12,96 |
| gef. | C 75,27 | H 8,86 | N 2,71 | |

### c) 2-(10-Isopropoxycarbonyldecyl)-glycinethylester

20,5 g (41,5 mmol) N-(Diphenylmethylen)-2-(10-isopropoxycarbonyldecyl)-glycinethylester werden in 200 ml Methanol gelöst und unter Zusatz von 2,1 g Pd auf Aktivkohle (10%) bei Normaldruck hydriert. Nach beendeter Wasserstoffaufnahme wird der Katalysator abfiltriert und der Rückstand zur Trockne eingedampft. Zur Reinigung wird das Öl chromatographiert.

Ausbeute: 12,9 g (94,3 % d. Th.) farbloses Öl.

| Analyse (bezogen auf lösungsmittelfreie Substanz): | | | | |
|---|---|---|---|---|
| ber. | C 65,62 | H 10,71 | N 4,25 | O 19,42 |
| gef. | C 65,53 | H 10,80 | N 4,09 | |

### d) N,N-Bis-{2-[N',N'-bis-(benzyloxycarbonylmethyl)]-amino]-ethyl}-2-(10-isopropoxycarbonyldecyl)-glycin-ethylester

12,7 g (38,5 mmol) 2-(10-lsopropoxycarbonyldecyl)-glycinethylester und 36,4 g (86,6 mmol) N,N-Bis-[(benzyloxycarbonyl)-methyl]-2-bromethylamin (M. Williams und H Rapoport, J. Org. Chem. 58, 1151 (1993)) werden in 40 ml Acetonitril vorgelegt und mit 35 ml 2 n Phosphatpufferlösung (pH 8,0) versetzt. Der Ansatz wird bei Raumtemperatur 20 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (4:1:0,01) chromatographiert. Die produkthaltigen Fraktionen werden im Vakuum eingedampft.

Ausbeute: 38,7 g (77 % d. Th.) farbloses Öl.

| Analyse (bezogen auf lösungsmittelfreie Substanz): | | | | |
|---|---|---|---|---|
| ber. | C 69,09 | H 7,70 | N 4,17 | O 19,04 |
| gef. | C 69,20 | H 7,78 | N 4,03 | |

### e) N,N-Bis-{2-[N',N'-bis-(carboxymethyl)]-amino]-ethyl}-2-(10-carboxydecyl)-glycin

33,6 g (33,3 mmol) N,N-Bis-{2-[N',N'-bis-(benzyloxycarbonylmethyl)]-amino]-ethyl}-2-(10-isopropoxycarbonyldecyl)-glycin-ethylester werden in 300 ml Methanol gelöst, mit 100 ml 2n Natronlauge versetzt und ca. 8 Stunden bei 60°C gerührt. Anschließend wird die Lösung mit konzentrierter Salzsäure auf pH 1 eingestellt und eingedampft; der Rückstand wird auf eine Säule mit stark saurem lonenaustauscher gegeben und mit wäßriger Ammoniaklösung eluiert. Die das Produkt enthaltenden Eluatfraktionen werden im Vakuum eingedampft und am Ölpumpenvakuum getrocknet.

Ausbeute: 15,8 g (82,1.% d Th.)

| Analyse (bezogen auf lösungsmittelfreie Substanz): | | | | |
|---|---|---|---|---|
| ber. | C 51,98 | H 7,50 | N 7,28 | O 33,24 |
| gef. | C 51,75 | H 7,66 | N 7,14 | |

### f) Hafniumkomplex des Dinatriumsalzes des N,N-Bis-{2-[N',N'-bis-(carboxymethyl)-amino]-ethyl}-2-(10-carboxydecyl)-glycin

15,3 g (26,5 mmol) N,N-Bis-{2-[N',N'-bis-(carboxymethyl)]-amino]-ethyl}-2-(10-carboxydecyl)-glycin werden in Wasser suspendiert und mit Hafniumhydroxid (D.J. Williams et al., J. Chem. Soc. Dalton Trans. 2475,1992) umgesetzt. Nach vollständiger Komplexierung neutralisiert man mit einnormaler Natronlauge, filtriert und lyophilisiert die Komplexlösung.

Ausbeute: 19,7 g (93,4 % d. Th.) farbloses Lyophilisat.

| Analyse (bezogen auf wasserfreie Substanz): | | | | | | |
|---|---|---|---|---|---|---|
| ber. | C 37,72 | H 4,68 | N 5,28 | O 24,12 | Hf 22,42 | Na 5,78 |
| gef. | C 37,67 | H 4,77 | N 5,16 | | Hf 22,30 | Na 5,46 |

### Beispiel 7

### Erbiumkomplex des Dinatriumsalzes von N,N-Bis-{2-[N',N'-bis-(carboxymethyl)]-amino]-ethyl}-2-methyl-DL-3-[(4-ethoxy)-phenyl]-alanin

Die Synthese erfolgt nach den in Beispiel 1 beschriebenen Bedingungen zur Darstellung des Dysprosiumkomplexes des Dinatriumsalzes von N,N-Bis-{2-[N',N'-bis-(carboxymethyl)]-amino]-ethyl}-L-3-[(4-ethoxy)-phenyl]-alanin, wobei anstelle von L-Tyrosinbenzylester-4-methylbenzolsulfonat α-Methyl-DL-p-tyrosinmethylester-Hydrochlorid (Fluka) als Startmaterial eingesetzt wird.

| Analyse (bezogen auf wasserfreie Substanz): | | | | | | |
|---|---|---|---|---|---|---|
| ber. | C 38,45 | H 4,03 | N 5,61 | O 23,47 | Er 22,31 | Na 6,13 |
| gef. | C 38.43 | H 4,22 | N 5,43 | | Er 22,25 | Na 6,19 |

### Pharmakologische Untersuchungen an der Ratte

Die Verbindung nach Beispiel 1 wird an der Ratte nach Aufnahme in die Leber ebenfalls überwiegend biliär ausgeschieden wie Gd-EOB-DTPA ((4S)-4-(4-Ethoxybenzyl)-3,6,9-tris(carboxylatomethyl)-3,6,9-triazaundecandisäure, Gadolinium-Komplex, Dinatriumsalz; vgl.: Schuhmann-Giampieri G., Investigative Radiology (1993) 28/8, 753-761).

In orientierenden Kreislaufuntersuchungen an narkotisierten Ratten wurden hämodynamische Parameter, wie Blutdruck, Gefäßwiderstand, Herzfrequenz und Kontraktilität des linken Ventrikels durch die Verbindung nach Beispiel 1 (Dosis berechnet auf 0,5 mmol Metall/kg Körpermasse, als Bolus intravenös verabreicht) während eines 45 minütigem Beobachtungszeitraumes anfangs leicht gesenkt (< -15 % gemessen gegenüber den Vorwerten), bei einem nur unmittelbar nach der Bolusgabe etwas erhöhten Herzminutenvolumen (+18 % gemessen gegenüber den Vorwerten) und enddiastolischen Druck (+4 mm Hg gemessen gegenüber den Vorwerten).

Gd-EOB-DTPA zeigt bei gleicher Dosierung an der gleichen Spezies nach intravenöser Bolusgabe stärker senkende Einflüsse auf den Blutdruck und den Gefäßwiderstand (-25 bis -30 % gemessen gegenüber den Vorwerten) im Zeitraum unmittelbar nach der Applikation bis 5 Minuten nach Applikation. Danach kommt es wie bei der Verbindung nach Beispiel 1 zur Normalisierung der Veränderungen.

Alle weiteren untersuchten hämodynamischen Parameter waren sowohl bei der Verbindung nach Beispiel 1 wie bei Gd-EOB-DTPA nur marginal vermindert (< -15 % gemessen gegenüber den Vorwerten) bzw. beim Herzminutenvolumen und enddiastolischem Druck marginal erhöht.

Die Verbindung nach Beispiel 1 ist somit im Vergleich zu Gd-EOB-DTPA ein (Leber-)Kontrastmittel mit verbesserter Herz-Kreislaufverträglichkeit.

## Patentansprüche

1. Metallkomplexe der allgemeinen Formel I 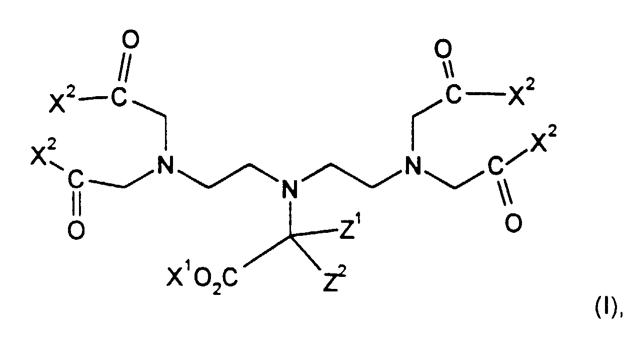 worin
X¹ unabhängig voneinander für ein Wasserstoffatom oder ein Metallionenäquivalent eines Elements der Ordnungszahlen 20 - 32, 39 - 51 oder 57 - 83 steht
X² unabhängig voneinander für eine Gruppe
O-X¹ mit X¹ in der oben angegebenen Bedeutung oder
N(R¹)R² worin
R¹, R² unabhängig voneinander für ein Wasserstoffatom oder für eine gesättigte oder ungesättigte, verzweigte oder geradkettige C₁-C₂₀-Kette steht, wobei die Kette oder Teile der Kette eine zyklische oder bizyklische Einheit formen können,
die durch null bis drei Sauerstoff- und/oder Schwefelatome und/oder null bis drei Sulfoxy- und/oder Sulfonogruppen unterbrochen ist und durch null bis sechs Phenyl-, Pyridyl-, R³S-, R³OOC- und/oder R³O-Gruppen substituiert ist, 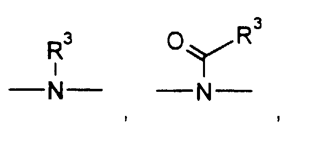 die weiterhin null bis drei 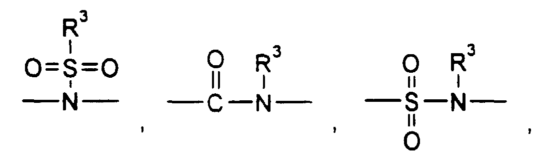 Carbonyl- und/oder Thiocarbonylgruppen enthält,
wobei gegebenenfalls vorhandene aromatische Gruppen ein- bis dreifach unabhängig voneinander durch R³O₂C-, 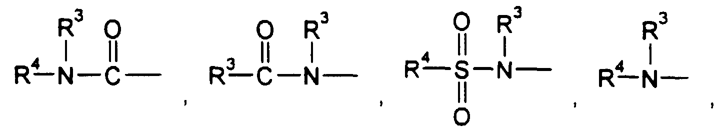 O₂N-, R³O- und/oder R⁴-Gruppen substituiert sein können
oder
R¹ und R² zusammen, unter Einbeziehung des gemeinsamen Amid-Stickstoffatomes einen vier- bis achtgliedrigen Ring bilden, der zwei weitere Sauerstoffatome und/oder zwei Carbonylgruppen enthalten kann,
Z¹ für eine gesättigte oder ungesättigte, verzweigte oder geradkettige C₁-C₂₀-Kette steht, wobei die Kette oder Teile der Kette eine zyklische oder bizyklische Einheit formen können,
die durch null bis drei Schwefelatome und/oder null bis drei Sulfoxy- und/oder Sulfonogruppen unterbrochen ist und
durch null bis sechs Phenyl-, Pyridyl-, R³S- und/oder R³OOC-Gruppen substituiert ist, 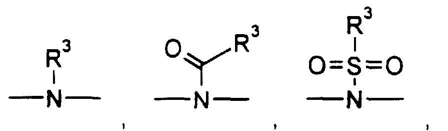 die weiterhin null bis drei 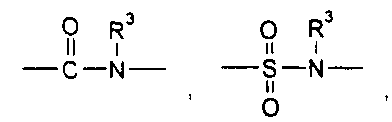 Carbonyl- und/oder Thiocarbonylgruppen enthält,
wobei gegebenenfalls vorhandene aromatische Gruppen ein- oder mehrfach unabhängig voneinander durch R³O₂C-, 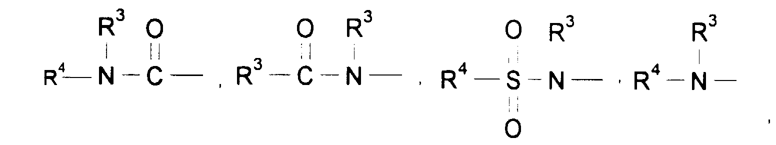 R³O- und/oder R⁴-Gruppen substituiert sein können,
Z² für ein Wasserstoffatom oder für eine gesättigte oder ungesättigte, verzweigte oder geradkettige C₁-C₂₀-Kette steht, wobei die Kette oder Teile der Kette eine zyklische oder bizyklische Einheit formen können,
die durch null bis drei Sauerstoff- und/oder Schwefelatome und/oder null bis drei Sulfoxy- und/oder Sulfonogruppen unterbrochen ist und
durch null bis sechs Phenyl-, Pyridyl-, R³S-, R³OOC- und/oder R³O-Gruppen substituiert ist, 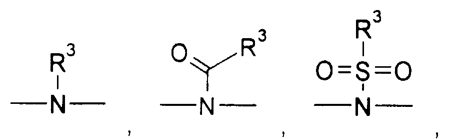 die weiterhin null bis drei 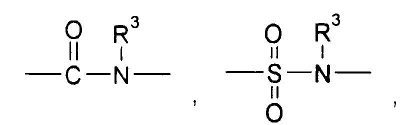 Carbonyl- und/oder Thiocarbonylgruppen enthält,
wobei gegebenenfalls vorhandene aromatische Gruppen ein- bis dreifach unabhängig voneinander durch R³O₂C-, 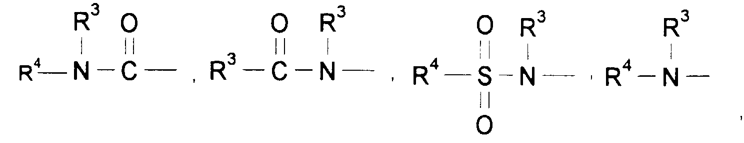 R³O- und/oder R³-Gruppen substituiert sein können,
R³ unabhängig voneinander für ein Wasserstoffatom, einen Phenylrest odereinen geradkettigen, verzweigten oder zyklischen C₁-C₆-Rest steht, der durch null bis zwei Sauerstoffatome und/oder null bis zwei Phenylengruppen unterbrochen ist und mit null bis drei HO-, HOOC- und/oder null bis zwei Phenylresten substituiert ist,
R⁴ unabhängig voneinander für einen Phenylrest oder einen geradkettigen, verzweigten oder zyklischen C₁-C₆-Rest steht, der durch null bis zwei Sauerstoffatome und/oder null bis zwei Phenylengruppen unterbrochen ist und mit null bis drei HO-, HOOC- und/oder null bis zwei Phenylresten substituiert ist,
wobei falls Z² für ein Wasserstoffatom steht der Rest Z¹ nicht für einen unsubstituierten C₆-C₁₀-Arylrest steht
oder Z¹ und Z² zusammen unter Einbeziehung des gemeinsamen α-Kohlenstoffatoms einen drei- bis achtgliedrigen Ring oder einen Bizyklus mit sieben bis 15 Kohlenstoffatomen formen,
wobei freie, zur Komplexierung nicht herangezogene Carbonsäuregruppen der erfindungsgemäßen Verbindungen der allgemeinen Formel I auch in Form ihrer Salze mit physiologisch verträglichen anorganischen und/oder organischen Kationen vorliegen können.

2. Verbindungen gemäß Anspruch 1, worin alle mit X² bezeichneten Gruppen für einen Rest O-X¹ stehen, worin X¹ die in Anspruch 1 genannte Bedeutung hat.

3. Verbindungen gemäß Anspruch 1, worin ein oder zwei der mit X² bezeichneten Gruppen für einen Rest N(R¹)R² stehen, worin R¹ und R² die in Anspruch 1 genannte Bedeutung haben.

4. Verbindungen gemäß Anspruch 1, worin als physiologisch verträgliche Kationen Natrium-, Calcium-, Magnesium-, Zink-, Meglumin-, Glucosamin-, Arginin-, Ornithin-, Lysin- und/oder Ethanolamin-ionen vorliegen.

5. Verbindungen gemäß Anspruch 1, worin Z¹ für einen C₆-C₂₀-Alkylrest steht.

6. Verbindungen gemäß Anspruch 1, worin Z¹ für einen Rest der Formel -(CH₂)ₙ-COOH steht, worin n für die Ziffern 1 bis 19 steht.

7. Verbindungen gemäß Anspruch 1, worin Z¹ für einen Rest der Formel 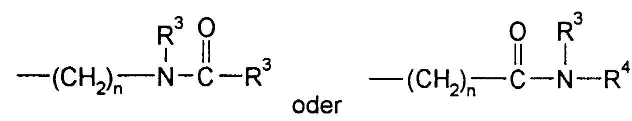 steht, worin n für die Ziffern 1 bis 19 steht und R³ sowie R⁴ die in Anspruch 1 genannte Bedeutung haben.

8. Verbindungen gemäß Anspruch 1, worin Z¹ für einen Rest der Formel -(CH₂)ₚ(C₆H₄)-C_{q}H_{(2q+1)} steht, worin p und q für die Ziffern 1 bis fünf stehen.

9. Verbindungen gemäß Anspruch 1, worin Z¹ für einen Rest der Formel -(CH₂)ₘ(C₆H₄)-R⁵ steht, worin R⁵ für einen Butyl-, Phenyl- oder Benzylrest und m für die Ziffern 1 bis 4 steht.

10. Verbindungen gemäß Anspruch 1, worin Z¹ für einen Rest der Formel -(CH₂)ₘ(C₆H₄)-O-R⁶ steht, worin R⁶ für ein Wasserstoffatom, einen C₁-C₆-Alkylrest, einen Carboxy-C₁-C₆-Alkylrest oder einen Phenyl- oder Benzylrest und m für die Ziffern 1 bis 4 steht.

11. Verbindungen gemäß Anspruch 1, worin Z¹ für einen Rest der Formel -CH₂(C₆H₄)-O-R⁷ steht, worin R⁷ für einen Methyl-, Ethyl-, n-Propyl-, iso-Propyl, n-Butyl-, iso-Butyl-, tert-Butyl-, Phenyl- oder Benzylrest steht und Z² für ein Wasserstoffatom steht.

12. Verbindungen gemäß Anspruch 1, worin Z¹ für einen Rest der Formel 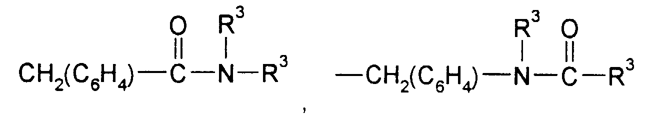 oder -CH₂(C₆H₄)-N(R³)R⁴ steht,
worin R³ und R⁴ die in Anspruch 1 genannten Bedeutungen haben.

13. Verbindungen gemäß Anspruch 1, worin Z¹ und Z² zusammen mit dem sie verbindenden Kohlenstoffatom einen fünf- bis achtgliedrigen Ring bilden .

14. Metallkomplexe gemäß Anspruch 1 wobei als Metall ein paramagnetisches Metall enthalten ist.

15. Metallkomplexe gemäß Anspruch 1 wobei das Metall radioaktiv ist.

16. Metallkomplexe gemäß Anspruch 1 wobei als Metall ein Metall der Lanthanoidenreihe enthalten ist.

17. Metallkomplexe gemäß Anspruch 1 wobei als Metall Gadolinium, Dysprosium, Holmium, Erbium, Terbium, Lutetium, Ytterbium, Wismut oder Hafnium enthalten ist.

18. Metallkomplexe gemäß Anspruch 1 wobei als Metall Mangan oder Eisen enthalten ist.

19. Metallkomplexe gemäß Anspruch 1 wobei als Metall Gallium, Indium oder Technetium enthalten ist.

20. Metallkomplexe gemäß Anspruch 1 wobei als Metall Kalzium oder Zink enthalten ist.

21. Dysprosiumkomplex von N,N-Bis-{2-[N',N'-bis-(carboxymethyl)]-amino]-ethyl}-L-3-[(4-methoxy)-phenyl]-alanin,
Dysprosiumkomplex von N,N-Bis-{2-[N',N'-bis-(carboxymethyl)]-amino]-ethyl}-L-3-[(4-ethoxy)-phenyl]-alanin,
Dysprosiumkomplex von N,N-Bis-{2-[N',N'-bis-(carboxymethyl)]amino]-ethyl}-L-3-[(4-propoxy)-phenyl]-alanin,
Dysprosiumkomplex von N,N-Bis-{2-[N',N'-bis-(carboxymethyl)]-amino]-ethyl}-L-3-[(4-butoxy)-phenyl]-alanin,
Dysprosiumkomplex von N,N-Bis-{2-[N',N'-bis-(carboxymethyl)]-amino]-ethyl}-L-3-[(4-benzyloxy)-phenyl]-alanin,
Gadoliniumkomplex von N,N-Bis-{2-[N',N'-bis-(carboxymethyl)]-amino]-ethyl}-L-3-[(4-methoxy)-phenyl]-alanin,
Gadoliniumkomplex von N,N-Bis-{2-[N',N'-bis-(carboxymethyl)]-amino]-ethyl}-L-3-[(4-ethoxy)-phenyl]-alanin,
Gadoliniumkomplex von N,N-Bis-{2-[N',N'-bis-(carboxymethyl)]-amino]-ethyl}-L-3-[(4-propoxy)-phenyl]-alanin,
Gadoliniumkomplex von N,N-Bis-{2-[N',N'-bis-(carboxymethyl)]-amino]-ethyl}-L-3-[(4-butoxy)-phenyl]-alanin,
Gadoliniumkomplex von N,N-Bis-{2-[N',N'-bis-(carboxymethyl)]-amino]-ethyl}-L-3-[(4-benzyloxy)-phenyl]-alanin,
Ytterbiumkomplex von N,N-Bis-{2-[N',N'-bis-(carboxymethyl)]-amino]-ethyl}-L-3-[(4-methoxy)-phenyl]-alanin,
Ytterbiumkomplex von N,N-Bis-{2-[N',N'-bis-(carboxymethyl)]-amino]-ethyl}-L-3-[(4-ethoxy)-phenyl]-alanin,
Ytterbiumkomplex von N,N-Bis-{2-[N',N'-bis-(carboxymethyl)]-amino]-ethyl}-L-3-[(4-propoxy)-phenyl]-alanin,
Ytterbiumkomplex von N,N-Bis-{2-[N',N'-bis-(carboxymethyl)]-amino]-ethyl}-L-3-[(4-butoxy)-phenyl]-alanin,
Ytterbiumkomplex von N,N-Bis-{2-[N',N'-bis-(carboxymethyl)]-amino]-ethyl}-L-3-[(4-benzyloxy)-phenyl]-alanin.

22. Wismutkomplex von N,N-Bis-{2-[N',N'-bis-(carboxymethyl)]-amino]-ethyl}-L-3-[(4-ethoxy)-phenyl]-alanin,
Mangankomplex von N,N-Bis-{2-[N',N'-bis-(carboxymethyl)]-amino]-ethyl}-L-3-[(4-ethoxy)-phenyl]-alanin,
Gadoliniumkomplex von N,N-Bis-[2-[N',N'-bis-(carboxymethyl)-amino]-ethyl]-α-aminolaurinsäure,
Ytterbiumkomplex von N_{α},N_{α}-Bis-[2-[N',N'-bis-(carboxymethyl]-amino]-ethyl]-N_{ε}-benzoyl-L-lysin,
Terbiumkomplex von N, N-Bis-{2-[N'-(carboxymethyl)]-N'-[(benzylcarbamoyl)-methyl]-amino]-ethyl}-L-glutaminsäure,
Gadoliniumkomplex von N,N-Bis-{2-[N',N'-bis-(carboxymethyl)]-amino]-ethyl}-3-[4-nitrophenyl]-alanin,
Ytterbiumkomplex vonTrinatriumsalzes von N,N-Bis-{2-[N',N'-bis-(carboxymethyl)]-amino]-ethyl}-3-[4-(carboxypropionylamino)-phenyl]-alanin,
Hafniumkomplex von N,N-Bis-{2-[N',N'-bis-(carboxymethyl)]-amino]-ethyl}-3-[4-(carboxypropionylamino)-phenyl]-alanin,
Hafniumkomplex von N,N-Bis-{2-[N',N'-bis-(carboxymethyl)-amino]-ethyl}-2-(10-carboxydecyl)-glycin,
Erbiumkomplex von N,N-Bis-{2-[N',N'-bis-(carboxymethyl)]-amino]-ethyl}-2-methyl-DL-3-[(4-ethoxy)-phenyl]-alanin.

23. Verfahren zur Herstellung von Verbindungen der allgemeinen Formel I 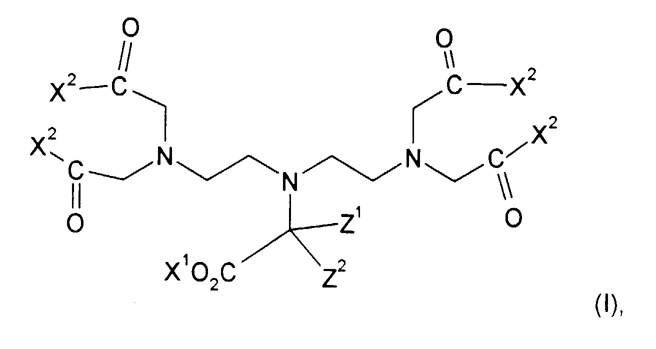 worin
X¹, X², Z¹ und Z² die in Anspruch 1 genannte Bedeutung haben, dadurch gekennzeichnet, daß eine Verbindung der allgemeinen Formel la 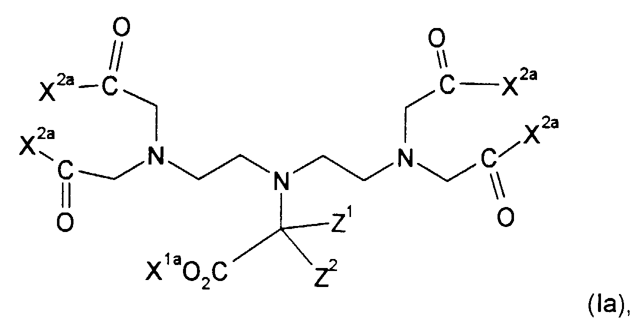 worin
Z¹ und Z² die in Anspruch 1 genannten Bedeutungen haben,
X^{1a} unabhängig voneinander für eine C₁- C₄-Alkyl- oder eine gegebenenfalls substituierte Benzylgruppe steht,
X^{2a} unabhängig voneinander für eine Gruppe
O-X^{1a} mit X^{1a} in der oben angegebenen Bedeutung oder
N(R¹)R² mit R¹, R² in der in Anspruch 1 genannten Bedeutung
steht,
durch Abspaltung der Säureschutzgruppen in Komplexbildner der allgemeinen Formel Ib überführt wird 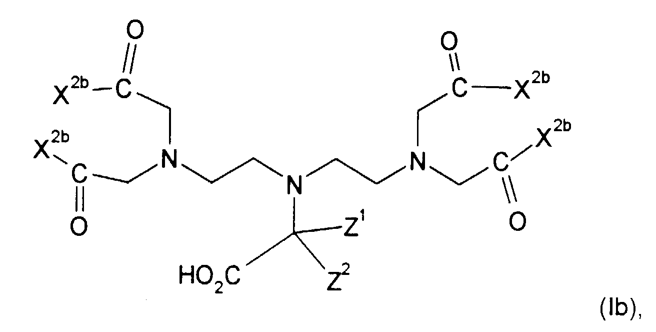 worin
Z¹ und Z² die in Anspruch 1 genannten Bedeutungen haben,
X^{2b} unabhängig voneinander für eine Gruppe
OH- oder
N(R¹)R² mit R¹, R² in der in Anspruch 1 genannten Bedeutung, stehen und der Komplexbildner anschließend durch Umsetzung mit einem Metalloxid oder einem Metallsalz eines Elements der Ordnungszahlen 20 - 32, 39 - 51 oder 57 - 83 in die erfindungsgemäßen Metallkomplexe überführt wird.

24. Pharmazeutische Mittel enthaltend mindestens eine physiologisch verträgliche Verbindung nach Anspruch 1, gegebenenfalls mit den in der Galenik üblichen Zusätzen.

25. Verwendung von mindestens einer physiologisch verträglichen Verbindung nach Anspruch 1, 21 oder 22 für die Herstellung von Mitteln für die Röntgendiagnostik.

26. Verwendung von mindestens einer physiologisch verträglichen Verbindung nach Anspruch 1, 21 oder 22 für die Herstellung von Mitteln für die NMR-Diagnostik.

27. Verwendung von mindestens einer physiologisch verträglichen Verbindung nach Anspruch 1, 21 oder 22 für die Herstellung von Mitteln für die Radiodiagnostik.

28. Verwendung von mindestens einer physiologisch verträglichen Verbindung nach Anspruch 1, 21 oder 22 für die Herstellung von Mitteln für die Röntgen-, NMR- und/oder Radiodiagnostik der Leber, der Galle und/oder der Gallengänge.

29. Verwendung von mindestens einer physiologisch verträglichen Verbindung nach Anspruch 1, 21 oder 22 für die Herstellung von Mitteln für die Radiotherapie.

30. Verwendung von mindestens einer physiologisch verträglichen Verbindung nach Anspruch 1 für die Herstellung von Mitteln zur Entfernung unerwünschter Schwermetalle aus dem Organismus.

31. Verwendung von mindestens einer physiologisch verträglichen Verbindung nach Anspruch 1 und 30 für die Herstellung von Mitteln zur Entfernung unerwünschter Schwermetalle aus der Leber.

## Claims

1. Metal complexes of the general formula I 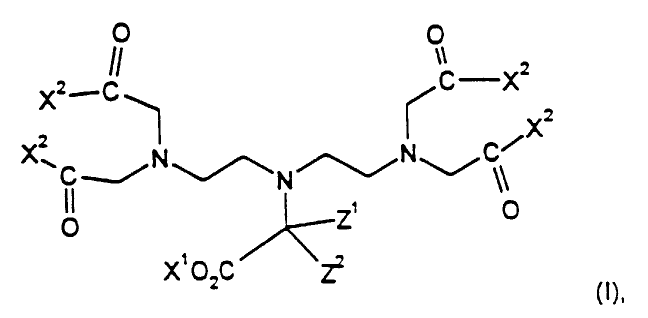 in which
X¹ is, independently of one another, a hydrogen atom or a metal ion equivalent of an element of atomic numbers 20-32, 39-51 or 57-83,
X² is, independently of one another, a group
O-X¹ with X¹ in the abovementioned meaning or
N(R¹)R² in which
R¹, R² is, independently of one another, a hydrogen atom or a saturated or unsaturated, branched or straight-chain C₁-C₂₀ chain, it being possible for the chain or parts of the chain to form a cyclic or bicyclic unit,
which is interrupted by zero to three oxygen and/or sulphur atoms and/or zero to three sulphoxy and/or sulphono groups, and
is substituted by zero to six phenyl, pyridyl, R³S, R³OOC and R³O groups,
which furthermore contains zero to three 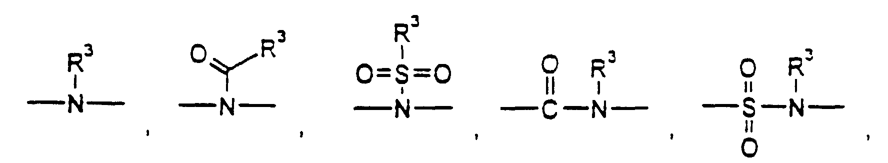 carbonyl and/or thiocarbonyl groups,
it being possible for aromatic groups which are present where appropriate to be substituted once to three times, independently of one another, by R³O₂C-, 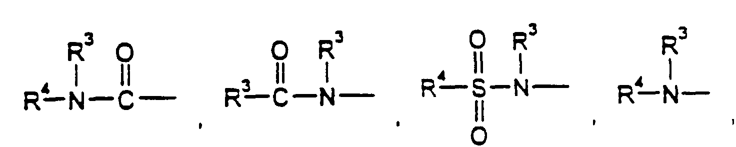 O₂N-, R³O- and/or R⁴ groups, or
R¹ and R² together form, with inclusion of the common amide nitrogen atom, a four- to eight-membered ring which may contain two other oxygen atoms and/or two carbonyl groups,
Z¹ is a saturated or unsaturated, branched or straight-chain C₁-C₂₀ chain, it being possible for the chain or parts of the chain to form a cyclic or bicyclic unit,
which is interrupted by zero to three sulphur atoms and/or zero to three sulphoxy and/or sulphono groups, and
is substituted by zero to six phenyl, pyridyl, R³S and/or R³OOC groups, which furthermore contains zero to three 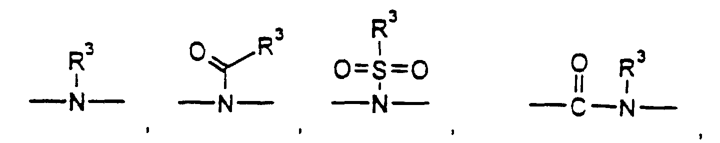 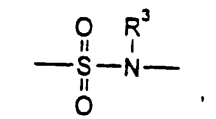 carbonyl and/or thiocarbonyl groups,
it being possible for aromatic groups which are present where appropriate to be substituted one or more times, independently of one another, by R³O₂C-, 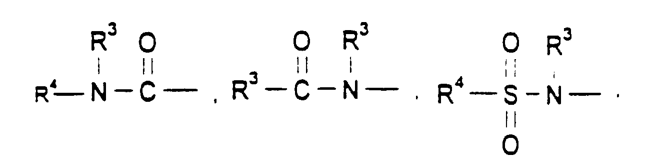 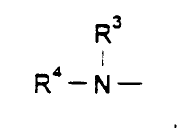 R³O- and/or R⁴ groups,
Z² is a hydrogen atom or a saturated or unsaturated, branched or straight-chain C₁-C₂₀ chain, it being possible for the chain or parts of the chain to form a cyclic or bicyclic unit,
which is interrupted by zero to three oxygen and/or sulphur atoms and/or zero to three sulphoxy and/or sulphono groups, and
is substituted by zero to six phenyl, pyridyl, R³S, R³OOC and/or R³O groups,
which furthermore contains zero to three 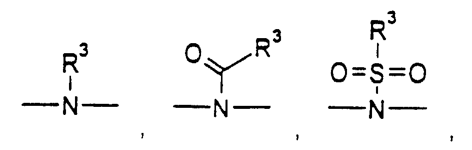 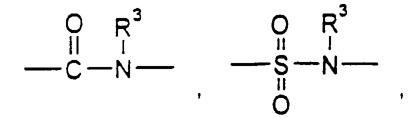 carbonyl and/or thiocarbonyl groups,
it being possible for aromatic groups which are present where appropriate to be substituted once to three times, independently of one another, by R³O₂C-, 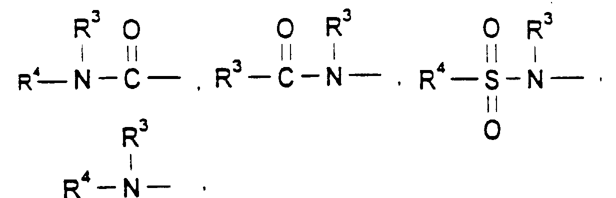 R³O- and/or R³ groups,
R³ is, independently of one another, a hydrogen atom, a phenyl radical or a straight-chain, branched or cyclic C₁-C₆ radical which is interrupted by zero to two oxygen atoms and/or zero to two phenylene groups, and is substituted by zero to three HO, HOOC and/or zero to two phenyl radicals,
R⁴ is, independently of one another, a phenyl radical or a straight-chain, branched or cyclic C₁-C₆ radical which is interrupted by zero to two oxygen atoms and/or zero to two phenylene groups, and is substituted by zero to three HO, HOOC and/or zero to two phenylene radicals,
where the radical Z¹ is not an unsubstituted C₆-C₂₀-aryl radical if Z² is a hydrogen atom,
or Z¹ and Z² together form, with inclusion of the common α carbon atom, a three- to eight-membered ring or a bicycle with seven to 15 carbon atoms,
it being possible for free carboxylic acid groups not used for the complexation in the compounds of the general formula I according to the invention also to be in the form of their salts with physiologically tolerated inorganic and/or organic cations.

2. Compounds according to Claim 1, in which all groups identified by X² are an O-X¹ radical in which X¹ has the meaning stated in Claim 1.

3. Compounds according to Claim 1, in which one or two of the groups identified by X² is an N(R¹)R² radical in which R¹ and R² have the meanings stated in Claim 1.

4. Compounds according to Claim 1, in which the physiologically tolerated cations present are sodium, calcium, magnesium, zinc, meglumine, glucosamine, arginine, ornithine, lysine and/or ethanolamine ions.

5. Compounds according to Claim 1, in which Z¹ is a C₆-C₂₀-alkyl radical.

6. Compounds according to Claim 1, in which Z¹ is a radical of the formula -(CH₂)ₙ-COOH in which n is the numbers 1 to 19.

7. Compounds according to Claim 1, in which Z¹ is a radical of the formula 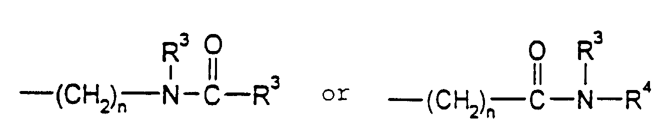 in which n is the numbers 1 to 19, and R³ and R⁴ have the meaning stated in Claim 1.

8. Compounds according to Claim 1, in which Z¹ is a radical of the formula -(CH₂)ₚ(C₆H₄)-C_{q}H_{(2q+1)} in which p and q are the numbers 1 to five.

9. Compounds according to Claim 1, in which Z¹ is a radical of the formula -(CH₂)ₘ(C₆H₄)-R⁵ in which R⁵ is a butyl, phenyl or benzyl radical and m is the numbers 1 to 4.

10. Compounds according to Claim 1, in which Z¹ is a radical of the formula -(CH₂)ₘ(C₆H₄)-O-R⁶ in which R⁶ is a hydrogen atom, a C₁-C₆-alkyl radical, a carboxy-C₁-C₆-alkyl radical or a phenyl or benzyl radical and m is the numbers 1 to 4.

11. Compounds according to Claim 1, in which Z¹ is a radical of the formula -CH₂(C₆H₄)-O-R⁷ in which R⁷ is a methyl, ethyl, n-propyl, isopropyl, n-butyl, isobutyl, tert-butyl, phenyl or benzyl radical and Z² is a hydrogen atom.

12. Compounds according to Claim 1, in which Z¹ is a radical of the formula 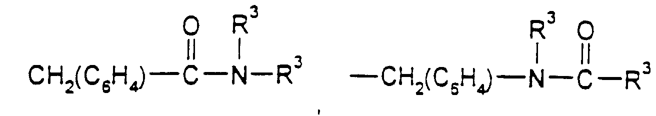 or -CH₂(C₆H₄)-N(R³)R⁴, in which R³ and R⁴ have the meanings stated in Claim 1.

13. Compounds according to Claim 1, in which Z¹ and Z² form, together with the carbon atom connecting them, a five- to eight-membered ring.

14. Metal complexes according to Claim 1 wherein a paramagnetic metal is present as metal.

15. Metal complexes according to Claim 1, wherein the metal is radioactive.

16. Metal complexes according to Claim 1, wherein a metal of the lanthanoid series is present as metal.

17. Metal complexes according to Claim 1, wherein gadolinium, dysprosium, holmium, erbium, terbium, lutetium, ytterbium, bismuth or hafnium is present as metal.

18. Metal complexes according to Claim 1, wherein manganese or iron is present as metal.

19. Metal complexes according to Claim 1, wherein gallium, indium or technetium is present as metal.

20. Metal complexes according to Claim 1, wherein calcium or zinc is present as metal.

21. Dysprosium complex of N,N-bis-{2-[N',N'-bis-(carboxymethyl)]-amino]-ethyl}-L-3-[(4-methoxy)-phenyl]-alanine,
dysprosium complex of N,N-bis-{2-[N',N'-bis-(carboxymethyl)]-amino]-ethyl}-L-3-[(4-ethoxy}-phenyl]-alanine,
dysprosium complex of N,N-bis-{2-[N',N'-bis-(carboxymethyl)]-amino]-ethyl}-L-3-[(4-propoxy)-phenyl]-alanine,
dysprosium complex of N,N-bis-{2-[N',N'-bis-(carboxymethyl)]-amino]-ethyl}-L-3-[(4-butoxy)-phenyl]-alanine,
dysprosium complex of N,N-bis-{2-[N',N'-bis-(carboxymethyl)]-amino]-ethyl}-L-3-[(4-benzyloxy)-phenyl]-alanine,
gadolinium complex of N,N-bis-{2-[N',N'-bis-(carboxymethyl)]-amino]-ethyl}-L-3-[(4-methoxy)-phenyl]-alanine,
gadolinium complex of N,N-bis-{2-[N',N'-bis(carboxymethyl)]-amino]-ethyl}-L-3-[(4-ethoxy}-phenyl]-alanine,
gadolinium complex of N,N-bis-{2-[N',N'-bis-(carboxymethyl)]-amino]-ethyl}-L-3-[(4-propoxy)-phenyl]-alanine,
gadolinium complex of N,N-bis-{2-[N',N'-bis-(carboxymethyl)]-amino]-ethyl}-L-3-[(4-butoxy)-phenyl]-alanine,
gadolinium complex of N,N-bis-{2-[N',N'-bis-(carboxymethyl)]-amino]-ethyl}-L-3-[(4-benzyloxy)-phenyl]-alanine,
ytterbium complex of N,N-bis-{2-[N',N'-bis-(carboxymethyl)]-amino]-ethyl}-L-3-[(4-methoxy)-phenyl]-alanine,
ytterbium complex of N,N-bis-{2-[N',N'-bis-(carboxymethyl)]-amino]-ethyl}-L-3-[(4-ethoxy)-phenyl]-alanine,
ytterbium complex of N,N-bis-{2-[N',N'-bis-(carboxymethyl)]-amino]-ethyl}-L-3-[(4-propoxy)-phenyl]-alanine,
ytterbium complex of N,N-bis-{2-[N',N'-bis-(carboxymethyl)]-amino]-ethyl}-L-3-[(4-butoxy)-phenyl]-alanine,
ytterbium complex of N,N-bis-{2-[N',N'-bis-(carboxymethyl)]-amino]-ethyl}-L-3-[(4-benzyloxy)-phenyl]-alanine.

22. Bismuth complex of N,N-bis-{2-[N',N'-bis-(carboxymethyl)]-amino]-ethyl}-L-3-[(4-ethoxy)-phenyl]alanine,
manganese complex of N,N-bis-{2-[N',N'-bis-(carboxymethyl)]-amino]-ethyl}-L-3-[(4-ethoxy}-phenyl]-alanine,
gadolinium complex of N,N-bis-[2-[N',N'-bis-(carboxymethyl)-amino]-ethyl]-α-aminolauric acid,
ytterbium complex of N_{α},N_{α}-bis-[2-[N',N'-bis-(carboxymethyl]-amino]-ethyl]-N_{ε}-benzoyl-L-lysine,
terbium complex of N,N-bis-{2-[N'-(carboxymethyl)]-N'-[(benzylcarbamoyl)-methyl]-amino]-ethyl}-L-glutamic acid,
gadolinium complex of N,N-bis-{2-[N',N'-bis-(carboxymethyl)]-amino]-ethyl}-3-[4-nitrophenyl]-alanine,
ytterbium complex of trisodium salt of N,N-bis-{2-[N',N'-bis-(carboxymethyl)]-amino]-ethyl}-3-[4-(carboxypropionylamino)-phenyl]-alanine,
hafnium complex of N,N-bis-{2-[N',N'-bis-(carboxy-methyl)]-amino]-ethyl}-3-[4-(carboxypropionylamino)-phenyl]-alanine,
hafnium complex of N,N-bis-{2-[N',N'-bis-(carboxy-methyl)-amino]-ethyl}-2-(10-carboxydecyl)-glycine,
erbium complex of N,N-bis-{2-[N',N'-bis-(carboxymethyl)]-amino]-ethyl}-2-methyl-DL-3-[(4-ethoxy)-phenyl]-alanine.

23. Process for preparing compounds of the general formula I 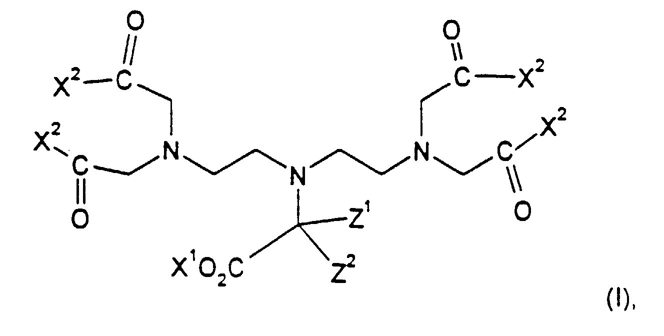 in which X¹, X², Z¹ and Z² have the meanings stated in Claim 1, characterized in that a compound of the general formula Ia 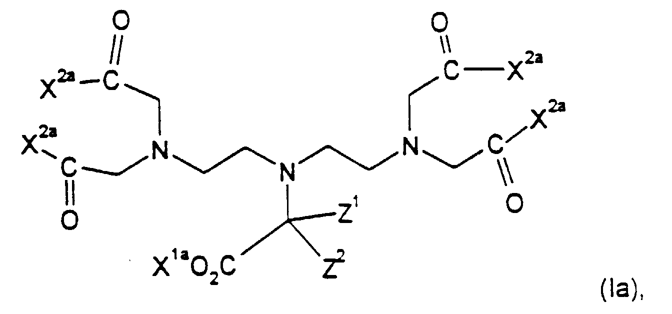 in which Z¹ and Z² have the meanings stated in Claim 1,
X^{1a} is, independently of one another, a C₁-C₄-alkyl or an optionally substituted benzyl group,
X^{2a} is, independently of one another, a group
O-X^{1a} with X^{1a} in the abovementioned meaning or
N(R¹)R² with R¹, R² in the meaning stated in Claim 1,
is converted, by eliminating the acid protective groups, into complexing agents of the general formula Ib 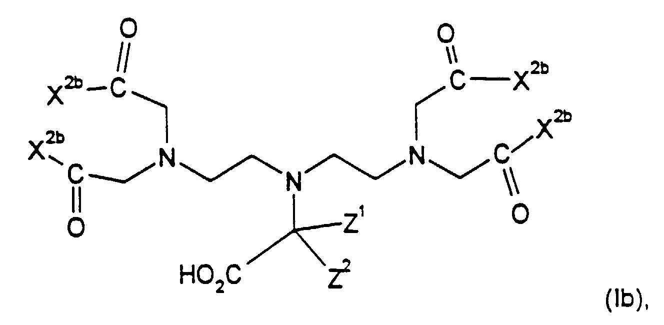 in which
Z¹ and Z² have the meanings stated in Claim 1, X^{2b} are, independently of one another, a group OH or
N(R¹)R² with R¹, R² in the meanings stated in Claim 1,
and the complexing agent is subsequently converted by reaction with a metal oxide or a metal salt of an element of atomic numbers 20-32, 39-51 or 57-83 into the metal complexes according to the invention.

24. Pharmaceutical compositions containing at least one physiologically tolerated compound according to Claim 1, where appropriate with the additions customary in pharmaceutical technology.

25. Use of at least one physiologically tolerated compound according to Claim 1, 21, or 22 for preparing compositions for X-ray diagnosis.

26. Use of at least one physiologically tolerated compound according to Claim 1, 21, or 22 for preparing compositions for NMR diagnosis.

27. Use of at least one physiologically tolerated compound according to Claim 1, 21, or 22 for preparing compositions for radionuclide diagnosis.

28. Use of at least one physiologically tolerated compound according to Claim 1, 21 or 22 for preparing compositions for X-ray, NMR and/or radionuclide diagnosis of the liver, the gall bladder and/or the bile ducts.

29. Use of at least one physiologically tolerated compound according to Claim 1, 21 or 22, for preparing compositions for radiotherapy.

30. Use of at least one physiologically tolerated compound according to Claim 1 for preparing compositions for removing unwanted heavy metals from the body.

31. Use of at least one physiologically tolerated compound according to Claim 1 for preparing compositions for removing unwanted heavy metals from the liver.

## Revendications

1. Complexes métalliques de formule générale I 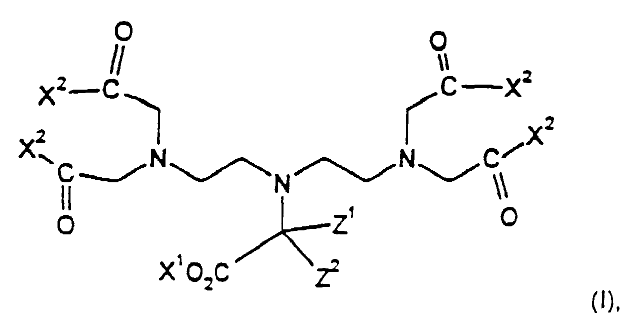 dans laquelle
X¹ désigne indépendamment un atome d'hydrogène ou un équivalent ion métallique d'un élément de numéros atomiques 20-32, 39-51 ou 57-83,
X² désigne indépendamment un groupe
O-X¹, X¹ ayant la signification indiquée ci-dessus
ou N(R¹)R², où
R¹, R² désignent, indépendamment l'un de l'autre un atome d'hydrogène ou une chaîne en C₁-C₂₀ ramifiée ou linéaire, saturée ou insaturée, la chaîne ou des parties de la chaîne pouvant former un motif cyclique ou bicyclique,
qui est interrompu par zéro à trois atomes d'oxygène et/ou de soufre et/ou zéro à trois groupes sulfoxy et/ou sulfono, et
est substitué par zéro à six groupes phényle, pyridyle, R³S, R³OOC et/ou R³O,
qui renferme en outre zéro à trois groupes 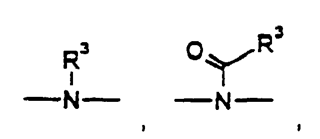 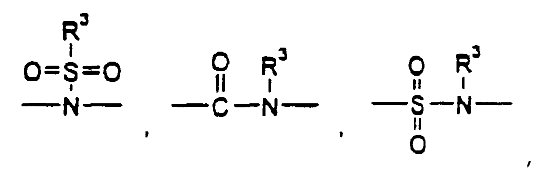 carbonyle et/ou thiocarbonyle,
les groupes aromatiques éventuellement présents pouvant être substitués, indépendamment les uns des autres, en une à trois positions par des groupes R³O₂C, 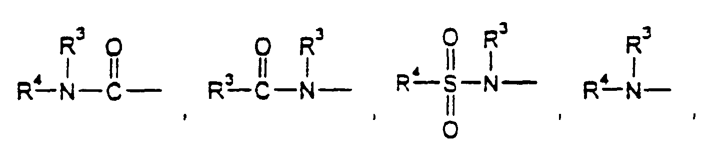 O₂N-, R³O- et/ou R⁴
ou R¹ et R² forment conjointement, à l'inclusion de l'atome d'azote d'amide commun, un cycle à quatre à huit chaînons, qui peut renfermer deux atomes d'oxygène supplémentaires et/ou deux groupes carbonyle,
Z¹ désigne une chaîne en C₁-C₂₀ ramifiée ou linéaire, saturée ou insaturée, la chaîne ou des parties de la chaîne pouvant former un motif cyclique ou bicyclique,
qui est interrompu par zéro à trois atomes de soufre et/ou zéro à trois groupes sulfoxy et/ou sulfono, et
est substitué par trois à six groupes phényle, pyridyle, R³S et/ou R³OOC,
qui renferme en outre zéro à trois groupes 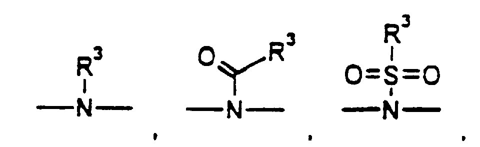 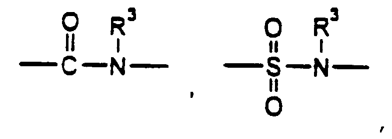 carbonyle et/ou thiocarbonyle,
les groupes aromatiques éventuellement présents pouvant être substitués indépendamment les uns des autres, en une ou plusieurs positions par des groupes R³O₂C, 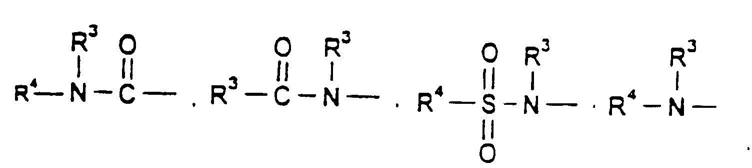 R³O- et/ou R⁴,
Z² désigne un atome d'hydrogène ou une chaîne en C₁-C₂₀ ramifiée ou linéaire, saturée ou insaturée, la chaîne ou des parties de la chaîne pouvant former un motif cyclique ou bicyclique,
qui est interrompu par zéro à trois atomes d'oxygène et/ou de soufre et/ou zéro à trois groupes sulfoxy et/ou sulfono, et
est substitué par trois à six groupes phényle, pyridyle, R³S, R³OOC et/ou R³O
qui renferme en outre zéro à trois groupes 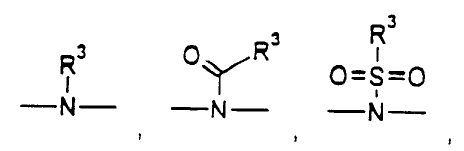 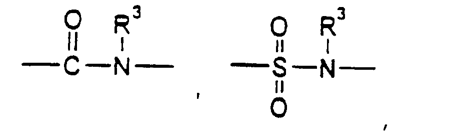 carbonyle et/ou thiocarbonyle,
les groupes aromatiques éventuellement présents pouvant être substitués indépendamment les uns des autres, en une ou plusieurs positions par des groupes R³O₂C, 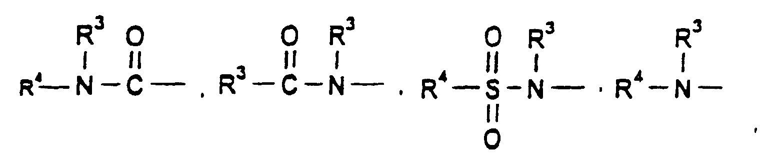 R³O- et/ou R³,
R³ désigne indépendamment un atome d'hydrogène, un radical phényle ou un radical en C₁-C₆ linéaire, ramifié ou cyclique, qui est interrompu par zéro à deux atomes d'oxygène et/ou zéro à deux groupes phénylène, et est substitué par zéro à trois radicaux HO, HOOC et/ou zéro à deux radicaux phényle,
R⁴ désigne indépendamment un radical phényle ou un radical en C₁-C₆ linéaire, ramifié ou cyclique, qui est interrompu par zéro à deux atomes d'oxygène et/ou zéro à deux groupes phénylène, et est substitué par zéro à trois radicaux HO, HOOC et/ou zéro à deux radicaux phényle,
où si Z² désigne un atome d'hydrogène, le radical Z¹ ne désigne pas un radical aryle en C₆-C₁₀ non substitué,
ou Z¹ et Z² forment conjointement, à l'inclusion de l'atome de carbone en α commun, un cycle à trois à huit chaînons ou un composé bicyclique ayant sept à quinze atomes de carbone,
des groupes acide carboxylique libres des composés selon l'invention de formule générale I, qui ne sont pas employés pour la complexation, pouvant également être présents sous forme de leurs sels avec des cations inorganiques et/ou organiques physiologiquement acceptables.

2. Composés selon la revendication 1, dans lesquels tous les groupes indiqués par X² désignent un radical O-X¹, où X¹ a la signification mentionnée à la revendication 1.

3. Composés selon la revendication 1, dans lesquels un ou deux des groupes indiqués par X² désignent un radical N(R¹)R², où R¹ et R² ont la signification mentionnée à la revendication 1.

4. Composés selon la revendication 1, dans lesquels les cations physiologiquement acceptables sont des ions du sodium, du calcium, du magnésium,, du zinc, de la méglumine, de la glucosamine, de l'arginine, de l'ornithine, de la lysine et/ou de l'éthanolamine.

5. Composés selon la revendication 1, dans lesquels Z¹ désigne un radical alkyle en C₆-C₂₀.

6. Composés selon la revendication 1, dans lesquels Z¹ désigne un radical de formule -(CH₂)ₙ-COOH, dans laquelle n désigne les chiffres 1 à 19.

7. Composés selon la revendication 1, dans lesquels Z¹ désigne un radical de formules 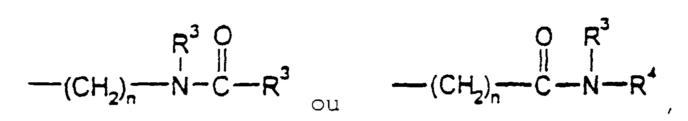 dans lesquelles n désigne les chiffres 1 à 19 et R³ ainsi que R⁴ ont la signification mentionnée à la revendication 1.

8. Composés selon la revendication 1, dans lesquels Z¹ désigne un radical de formule -(CH₂)ₚ(C₆H₄)-C_{q}H_{(2q+1)}, dans laquelle p et q désigne les chiffres 1 à cinq.

9. Composés selon la revendication 1, dans lesquels Z¹ désigne un radical de formule -(CH₂)ₘ(C₆H₄)-R⁵, dans laquelle R⁵ désigne un radical butyle, phényle ou benzyle et m désigne les chiffres 1 à 4.

10. Composés selon la revendication 1, dans lesquels Z¹ désigne un radical de formule -(CH₂)ₘ(C₆H₄)-O-R⁶, dans laquelle R⁶ désigne un atome d'hydrogène, un radical alkyle en C₁-C₆, un radical carboxy-C₁-C₆-alkyle ou un radical phényle ou benzyle, et m désigne les chiffres 1 à 4.

11. Composés selon la revendication 1, dans lesquels Z¹ désigne un radical de formule -CH₂(C₆H₄)-O-R⁷, dans laquelle R⁷ désigne un radical méthyle, éthyle, n-propyle, isopropyle, n-butyle, isobutyle, tert-butyle, phényle ou benzyle et Z² désigne un atome d'hydrogène.

12. Composés selon la revendication 1, dans lesquels Z¹ désigne un radical de formules 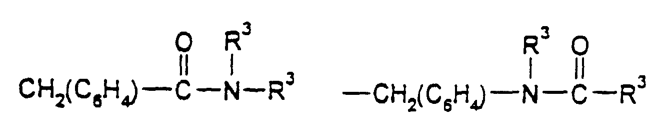 ou -CH₂(C₆H₄)-N(R³)R⁴, dans lesquelles R³ et R⁴ ont la signification mentionnée à la revendication 1.

13. Composés selon la revendication 1, dans lesquels Z¹ et Z² forment conjointement avec l'atome de carbone qui les lie, un cycle à cinq à huit chaînons.

14. Complexes métalliques selon la revendication 1, dans lesquels le métal présent est un métal paramagnétique.

15. Complexes métalliques selon la revendication 1, dans lesquels le métal est radioactif.

16. complexes métalliques selon la revendication 1, dans lesquels le métal présent est un métal de la série des lanthanides.

17. Complexes métalliques selon la revendication 1, dans lesquels le métal présent est le gadolinium, le dysprosium, le holmium, l'erbium, le terbium, le lutécium, l'ytterbium, le bismuth ou le hafnium.

18. Complexes métalliques selon la revendication 1, dans lesquels le métal présent est le manganèse ou le fer.

19. Complexes métalliques selon la revendication 1, dans lesquels le métal présent est le gallium, l'indium ou le technétium.

20. Complexes métalliques selon la revendication 1, dans lesquels le métal présent est le calcium ou le zinc.

21. Complexe au dysprosium de la N,N-bis-{2-[N'-N'-bis-(carboxyméthyl)]-amino-éthyl}-L-3-[(4-méthoxy)-phényl]-alanine,
complexe au dysprosium de la N,N-bis-{2-[N'-N'-bis-(carboxyméthyl)]-amino-éthyl}-L-3-[(4-éthoxy)-phényl]-alanine,
complexe au dysprosium de la N,N-bis-{2-[N'-N'-bis-(carboxyméthyl)]-amino-éthyl}-L-3-[(4-propoxy)-phényl]-alanine,
complexe au dysprosium de la N,N-bis-{2-[N'-N'-bis-(carboxyméthyl)]-amino-éthyl}-L-3-[(4-butoxy)-phényl]-alanine,
complexe au dysprosium de la N,N-bis-{2-[N'-N'-bis-(carboxyméthyl)]-amino-éthyl}-L-3-[(4-benzyloxy)-phényl]-alanine,
complexe au gadolinium de la N,N-bis-{2-[N'-N'-bis-(carboxyméthyl)]-amino-éthyl}-L-3-[(4-méthoxy)-phényl]-alanine,
complexe au gadolinium de la N,N-bis-{2-[N'-N'-bis-(carboxyméthyl)]-amino-éthyl}-L-3-[(4-éthoxy)-phényl]-alanine,
complexe au gadolinium de la N,N-bis-{2-[N'-N'-bis-(carboxyméthyl)]-amino-éthyl}-L-3-[(4-propoxy)-phényl]-alanine,
complexe au gadolinium de la N,N-bis-{2-[N'-N'-bis-(carboxyméthyl)]-amino-éthyl}-L-3-[(4-butoxy)-phényl]-alanine,
complexe au gadolinium de la N,N-bis-{2-[N'-N'-bis-(carboxyméthyl)]-amino-éthyl}-L-3-[(4-benzyloxy)-phényl]-alanine,
complexe à l'ytterbium de la N,N-bis-{2-[N'-N'-bis-(carboxyméthyl)]-amino-éthyl}-L-3-[(4-méthoxy)-phényl]-alanine,
complexe à l'ytterbium de la N,N-bis-{2-[N'-N'-bis-(carboxyméthyl)]-amino-éthyl}-L-3-[(4-éthoxy)-phényl]-alanine,
complexe à l'ytterbium de la N,N-bis-{2-[N'-N'-bis-(carboxyméthyl)]-amino-éthyl}-L-3-[(4-propoxy)-phényl]-alanine,
complexe à l'ytterbium de la N,N-bis-{2-[N'-N'-bis-(carboxyméthyl)]-amino-éthyl}-L-3-[(4-butoxy)-phényl]-alanine,
complexe à l'ytterbium de la N,N-bis-{2-[N'-N'-bis-(carboxyméthyl)]-amino-éthyl}-L-3-[(4-benzyloxy)-phényl]-alanine.

22. Complexe au bismuth de la N,N-bis-{2-[N'-N'-bis-(carboxyméthyl)]-amino-éthyl}-L-3-[(4-éthoxy)-phényl]-alanine,
complexe au manganèse de la N,N-bis-{2-[N'-N'-bis-(carboxyméthyl)]-amino-éthyl}-L-3-[(4-éthoxy)-phényl]-alanine,
complexe au gadolinium de l'acide N,N-bis-[2-[N'-N'-bis-(carboxyméthyl)]-amino-éthyl]-α-laurique,
complexe à l'ytterbium de la N_{α},N_{α}-bis-[2-[N'-N'-bis-(carboxyméthyl]-amino]-éthyl]-N_{ε}-benzoyle-L-lysine,
complexe au terbium de l'acide N,N-bis-{2-[N'-(carboxyméthyl)]-N'-[(benzylcarbamoyl)-méthyl]-amino-éthyl}-L-glutaminique,
complexe au gadolinium de la N,N-bis-{2-[N'-N'-bis-(carboxyméthyl)]-amino-éthyl}-3-[4-nitrophényl]alanine,
complexe à l'ytterbium du sel trisodique de la N,N-bis-{2-[N',N'-bis-(carboxyméthyl)]-amino-éthyl]}-3-[4-(carboxypropionylamino)-phényl]-alanine,
complexe au hafnium de la N,N-bis-{2-[N',N'-bis-(carboxyméthyl)]-amino-éthyl]}-3-[4-(carboxypropionylamino)-phényl]-alanine,
complexe au hafnium de la N,N-bis-{2-[N',N'-bis-(carboxyméthyl)]-amino-éthyl}-2-(10-carboxydécyl)-glycine,
complexe à l'erbium de la N,N-bis-{2-[N',N'-bis-(carboxyméthyl)]-amino-éthyl}-2-méthyl-DL-3-[(4-éthoxy)-phényl]-alanine,

23. Procédé de préparation de composés de formule générale I 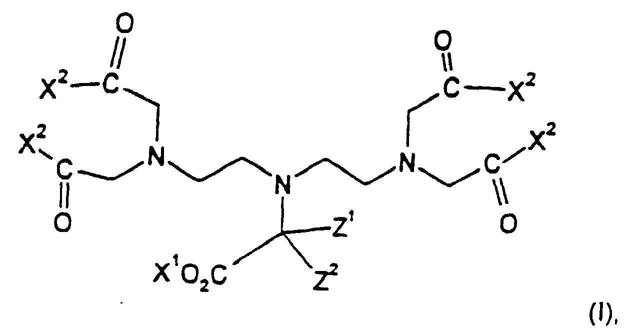 dans laquelle
X¹, X², Z¹ et Z² ont la signification mentionnée à la revendication 1, caractérisé en ce qu'un composé de formule générale Ia 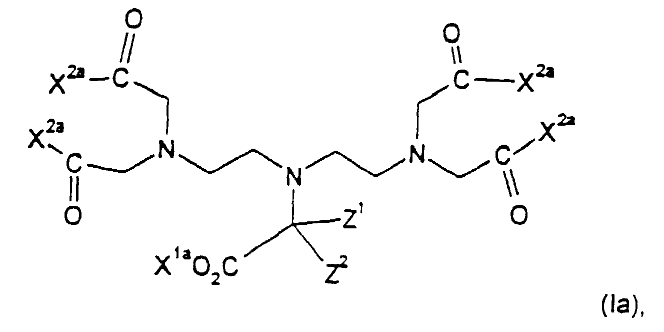 dans laquelle
Z¹ et Z² ont les significations mentionnées à la revendication 1,
X^{1a} désigne indépendamment un groupe alkyle en C₁-C₄ ou un groupe benzyle éventuellement substitué,
X^{2a} désigne indépendamment un groupe
O-X^{1a}, X^{1a} ayant la signification indiquée ci-dessus
ou N(R¹)R², R¹ et R² ayant la signification mentionnée à la revendication 1,
est transformé, par élimination des groupes protecteurs d'acide, en un agent complexant de formule générale Ib 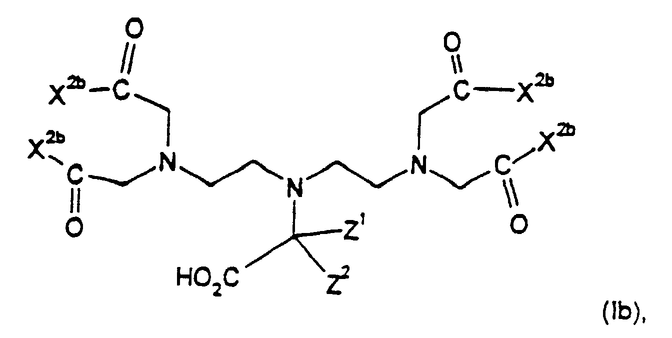 dans laquelle
Z¹ et Z² ont les significations mentionnées à la revendication 1,
X^{2b} désigne indépendamment un groupe
OH ou
N(R¹)R², R¹ et R² ayant la signification mentionnée à la revendication 1,
et l'agent complexant est ensuite transformé en complexes métalliques selon l'invention par réaction avec un oxyde métallique ou un sel métallique d'un élément de numéros atomiques 20-32, 39-51 ou 57-83.

24. Compositions pharmaceutiques contenant au moins un composé physiologiquement acceptable selon la revendication 1, éventuellement avec des additifs habituels dans le domaine galénique.

25. Utilisation d'au moins un composé physiologiquement acceptable selon la revendication 1, 21 ou 22, pour la préparation de compositions destinées au diagnostic par rayons X.

26. Utilisation d'au moins un composé physiologiquement acceptable selon la revendication 1, 21 ou 22, pour la préparation de compositions destinées au diagnostic par RMN.

27. Utilisation d'au moins un composé physiologiquement acceptable selon la revendication 1, 21 ou 22, pour la préparation de compositions destinées au radiodiagnostic.

28. Utilisation d'au moins un composé physiologiquement acceptable selon la revendication 1, 21 ou 22, pour la préparation de compositions destinées au diagnostic par rayons X, par RMN et/ou au radiodiagnostic du foie, de la bile et/ou des canaux biliaires.

29. Utilisation d'au moins un composé physiologiquement acceptable selon la revendication 1, 21 ou 22, pour la préparation de compositions destinées à la radiothérapie.

30. Utilisation d'au moins un composé physiologiquement acceptable selon la revendication 1, pour la préparation de compositions destinées à l'élimination de métaux lourds indésirables de l'organisme.

31. Utilisation d'au moins un composé physiologiquement acceptable selon les revendications 1 et 30, pour la préparation de compositions destinées à l'élimination de métaux lourds indésirables du foie.
